# EUROPEAN PATENT APPLICATION

(11) **EP 4 174 176 A1**
(43) Date of publication of application: **03.05.2023**
(21) Application number: 21382971.6
(22) Date of filing: 27.10.2021
(51) Int. Cl.: C12N 15/113

(54) **ANTAGONIST OF THE MIRNA-106B-5P TO TREAT OR PREVENT THE CARDIOTOXICITY THAT IS DEVELOPED IN ONCOLOGIC PATIENTS AFTER RECEIVING ANTHRACYCLINE-BASED CHEMOTHERAPY**

(71) Applicant: Universidad de Murcia, 30100 Espinardo (ES)
(72) Inventor: PASCUAL FIGAL, Domingo Andrés, 30120 Murcia (ES); LAX PÉREZ, Antonio Manuel, 30120 Murcia (ES); ASENSIO LÓPEZ, María del Carmen, 30120 Murcia (ES)
(74) Representative: Hoffmann Eitle

(57) **Abstract**

It is herein demonstrated that an increase in Dox-induced miRNA-106b-5p (miR-106b) is able to down-regulate PR55α subunit, which leds to a decrease both in PP2A activity as well as an accumulation of the phosphorylated HDAC4 protein in cytosol. Anti-miRNA-106b-5p (miR-106b) therapy is thus able to prevent Dox-induced PR55α degradation, facilitate the HDAC4 dephosphorylation by PP2A, and increase HDAC4-YY1 interaction, resulting in a decreased in sST2 expression and release by the LV myocardium. Further, and for first time, it has identified the role that sST2 isoform plays in Dox-induced CTX.

## Description

### TECHNICAL FIELD

The present invention is directed to the medical field, more particularly to the treatment of the cardiotoxicity that is developed in oncologic patients after receiving anthracycline-based chemotherapy by using antagonist of the miRNA-106b-5p.

### BACKGROUND ART

Among anticancer drugs, anthracycline family members such as doxorubicin (Dox) are known to induce cumulative dose-dependent cardiotoxicity (CTX) and heart failure (HF) . The quality of life of these patients is poor, resulting in significant morbidity and mortality. To date, there is no definitive and effective treatment to prevent anthracyclines-induced CTX. Indeed, these patients are treated similarly to non-oncologic patients. Overall, these are suboptimal and non-specific treatments. In the field of adverse remodeling after myocardial infarction (MI), studies on the modulation of the poor prognostic soluble ST2 (sST2) marker have had a profound impact on the management of patients with ischemic HF. In fact, up to the date, there was currently no treatment able to specifically lower sST2 levels. In a high percentage of patients, no matter what treatment they receive, their sST2 levels does not decrease at discharge. They are patients with worse prognostic and higher index of death. We have recently identified Yin-yang 1 (YY1) as a transcription factor that is able to up-regulate sST2 expression and HDAC4 as its necessary co-repressor, since it is able to block the up-regulated expression of sST2 in the setting of MI10. Whether YY1/HDAC4 axis is related to Dox-induced cardiac dysfunction in oncologic patients, the role of circulating sST2 and even the associated regulatory molecular mechanism, were unknow thus far.

MicroRNAs (miRNAs) are small, non-coding RNA molecules that exert their regulatory function either by translational repression or by mRNA degradation of their targets. In this invention, we focus on miRNA-106b-5p (miR-106b), which has been demonstrated to be aberrantly expressed in different human solid malignancies. According to these studies, miR-106b upregulation is frequent in tumor progression, and such upregulation is involved in the development of invasiveness and metastasis or melanoma growth. Interestingly, it has been reported that its suppression is able to induce cell cycle arrest and results in blocked growth of melanoma cells. In a study published in 2016, using an experimental animal model with cerebral ischemia/reperfusion (I/R) injury, authors demonstrated that its systemic silencing was able to ameliorate cerebral damage via blocking oxidative stress (for a review, see Li et al., An Antagomir to MicroRNA-106b-5p Ameliorates Cerebral Ischemia and Reperfusion Injury in Rats Via Inhibiting Apoptosis and Oxidative Stress). A pathological phenomenon, that according to other studies carried out recently, is activated by sST2 protein. In this invention, we demonstrate that miR-106b is related to Dox-induced CTX via its implication in the YY1/HDAC4 axis which over-expresses sST2 producing the cardiac dysfunction induced by the anthracycline. In particular, the data provided in the present invention demonstrates the role of the HDAC4/YY1 axis on Dox-induced CTX and its molecular regulation by miR-106b, which leads to an increase in sST2 expression and cardiac dysfunction. Our results demonstrate that the functional and systemic miR-106b blocking before, during or after starting anthracycline treatment is, for the first time, demonstrated to be able to treat and or prevent anthracycline induced CTX, leading to the prevention of the development of HF.

### BRIEF DESCRIPTION OF THE DRAWINGS

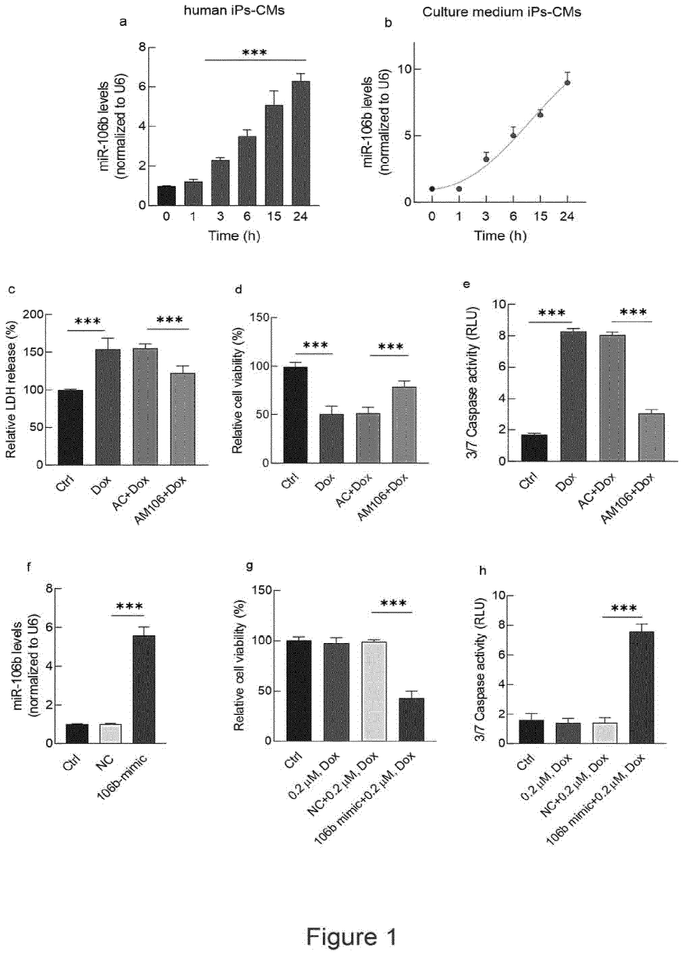

Fig 1. MiR-106b promotes Dox-induced myocardial apoptosis. (a) MiR-106b levels in hiPsCMs. (b) miR-106b levels in cell supernatant from hiPsCMs. (c) LDH levels in cell supernatant obtained from hiPsCMs. (d) Relative cell viability. (e) Caspase 3/7 activity. (f) miR-106b levels. (g) Relative cell viability. (h) Caspase 3/7 activity. All quantifications are derived from n = 5 independent assays/group, and PCR experiments were performed with 2 replicates each. All quantitative data are presented as mean ± SEM. ***p < 0.001 determined by 2-way ANOVA followed by Bonferroni post hoc test. Abbreviations: U6: U6 small nucleolar RNA; Ctrl: control; AC: antimiR-control; AM: antimiR; and NC: negative control.

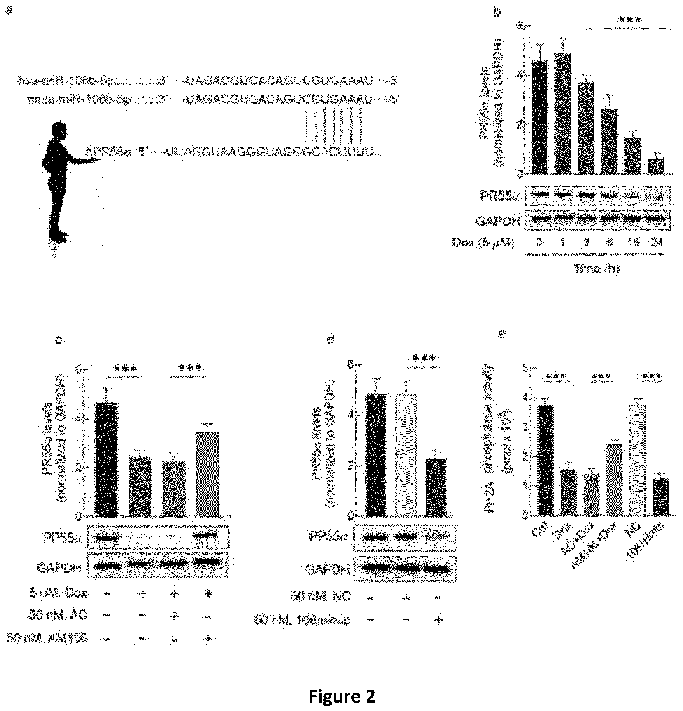

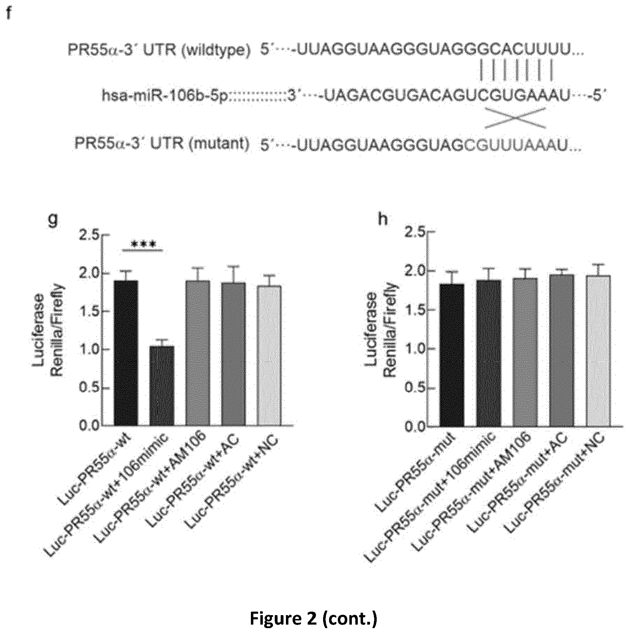

Fig 2. PR55α regulatory subunit is a direct target of miR-106b. (a) Binding sequence of hsa miR-106b and mmu-miR-106b within PR55α mRNA. (b) Representative Western blot image and analysis of PR55α expression levels in cells treated with Dox for different time periods. (c-d) Representative Western blot and analysis of PR55α expression levels in hiPsCMs subjected to different experimental treatments. (e) PP2A phosphatase activity in hiPsCMs subjected to different treatments. (f) Sequences and position of PR55α containing the wild-type and mutant binding site of miR-106b that was cloned into the luciferase vector. (g-h) Luciferase assay. Quantifications are derived from n = 5 independent assays/group, PCR experiments were performed with 2 replicates each. All quantitative data are presented as mean ± SEM. ***p < 0.001 determined by 2-way ANOVA followed by Bonferroni post hoc test. Abbreviations: GAPDH: Glyceraldehyde 3-phosphate dehydrogenase; Dox: doxorubicin. Other abbreviations previously described.

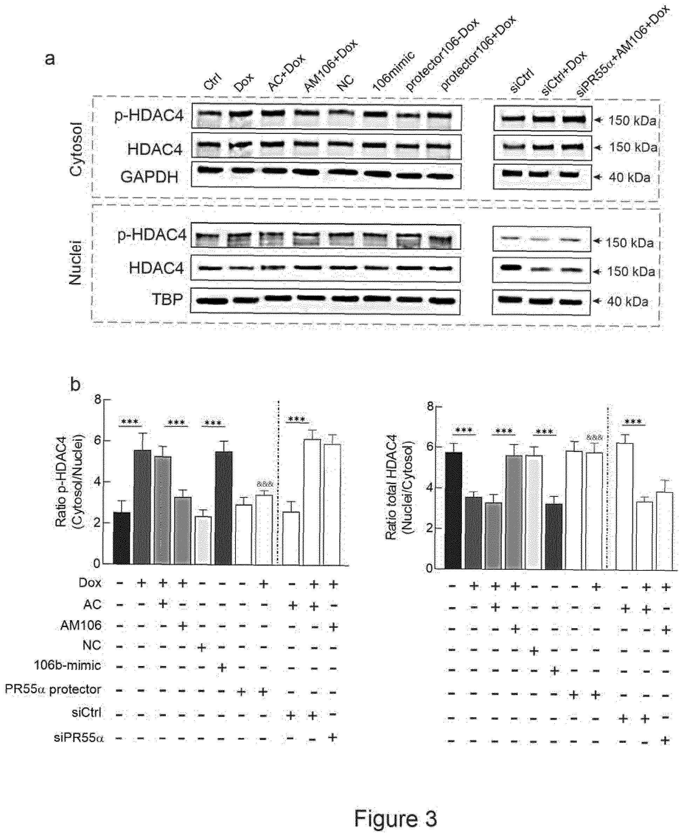

Fig 3. AntimiR-106b therapy prevents Dox-induced accumulation of phosphorylated HDAC4 in the cytosol. (a) Representative Western blot of the levels of phosphorylated accumulation and total HDAC4 levels in the cytosol and nucleus in cells under different treatments. (b) Densitometric analysis calculated as the ratio of accumulated levels of phosphorylated HDAC4 in the cytosol to accumulated levels in the nucleus and total HDAC4 distribution. All quantifications are derived from n = 5 independent assays/group. All quantitative data are presented as mean ± SEM. ***p < 0.001 determined by two-way ANOVA followed by Bonferroni post hoc test. Abbreviations: TBP: TATA element-binding protein. Other abbreviations previously described

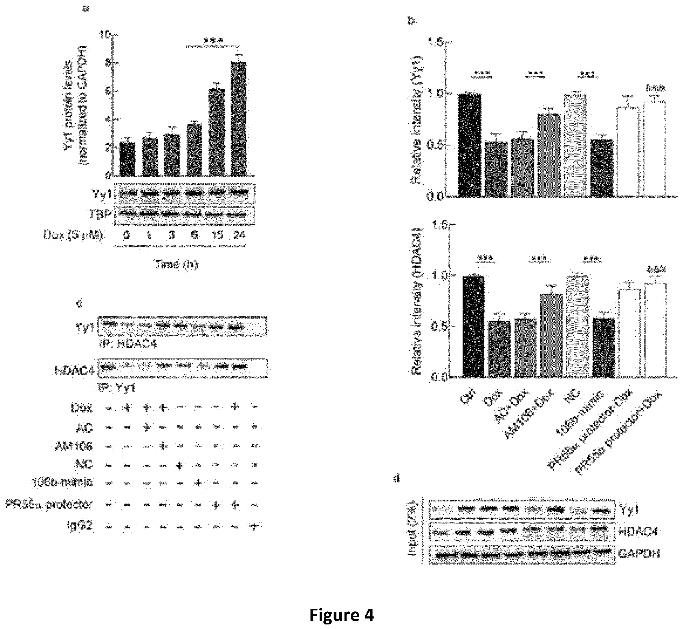

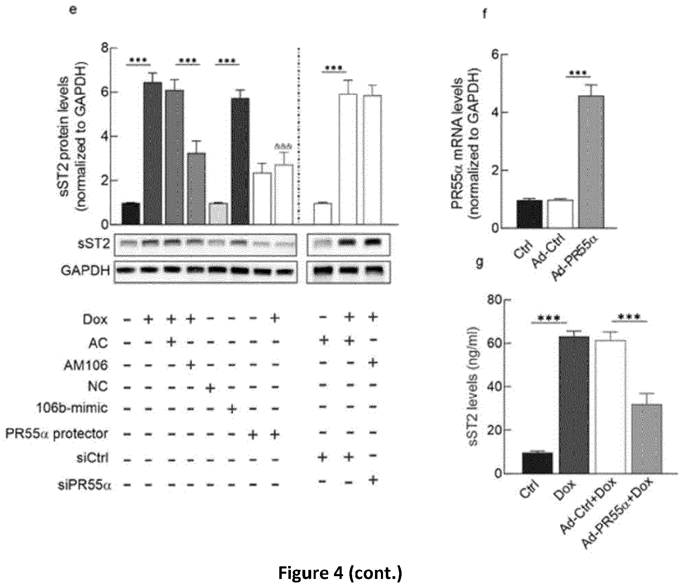

Fig 4. Anti-miR-106b therapy promotes HDAC4-YY1 interaction and prevents Dox-induced sST2 expression and secretion. (a) Representative Western blot and analysis of YY1 expression levels in hiPsCMs subjected to Dox treatment for different time periods. (b-d) Reciprocal co immunoprecipitation assays, representative western blot images and HDAC4/YY1 interaction analysis in nuclear fractions of hiPsCMs subjected to different treatments. (e) Representative western blot and analysis of sST2 expression levels in hiPsCMs under different treatments. (f) PR55α mRNA levels. (g) sST2 levels in supernatant obtained from hiPsCMs. All quantifications are derived from n = 5 independent assays/group, PCR experiments were performed with 2 replicates each. All quantitative data are presented as mean ± SEM. ***p < 0.001 determined by 2-way ANOVA followed by Bonferroni post hoc test.

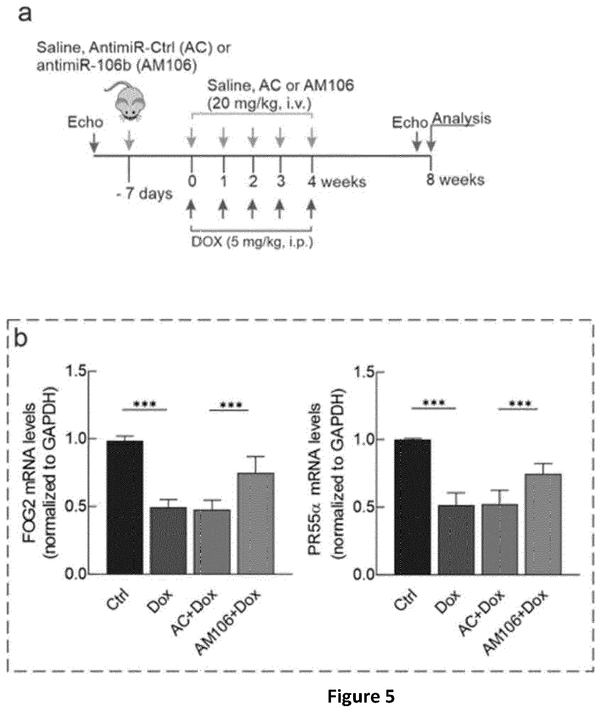

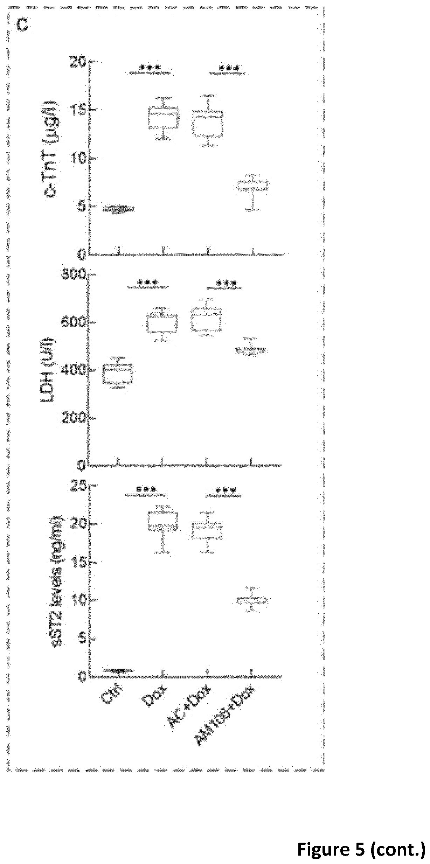

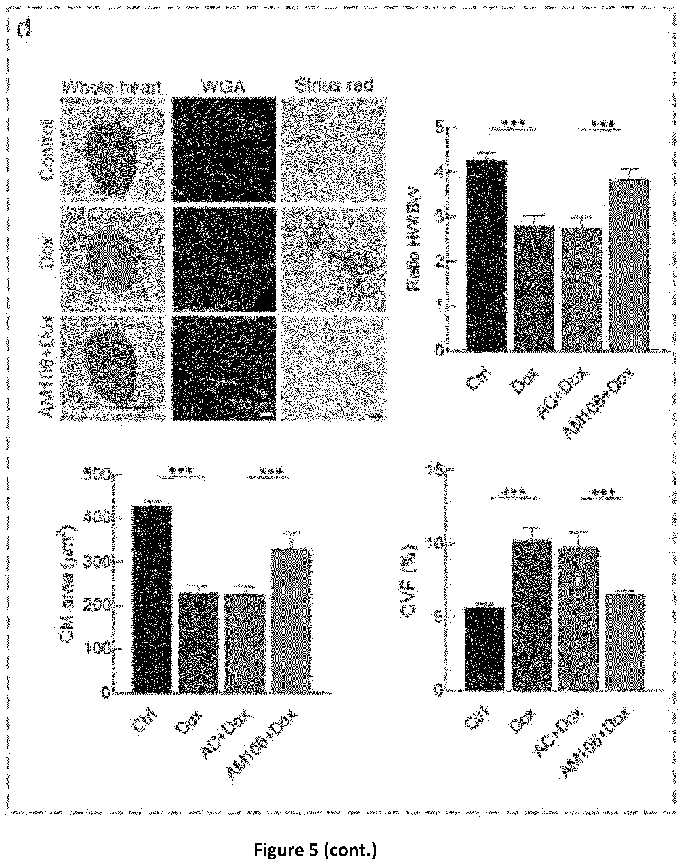

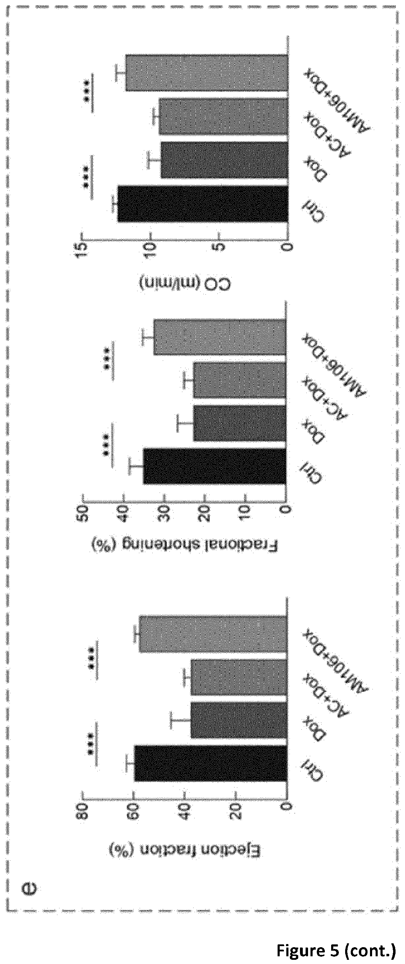

Fig 5. Anti-miR-106b therapy prevents Dox-induced CTX in mice. (a) Design of experimental study. (b) Levels of FOG2 and PR55α mRNA. (c) Plasma levels of c-TnT, LDH, and sST2. (d) Top-left panel: Left column, representative images of whole hearts under different treatments-scale bar: 0.5 cm; Middle column, micrographs obtained from left ventricle of mice subjected to different treatments and labelled with WGA staining-scale bar: 100 µm; Right column, micrographs obtained from left ventricle of mice subjected to different treatments and stained with Sirius red-scale bar: 100 µm. Top-right panel: ratio of heart weight to animal weight. Bottom-left panel: cardiomyocyte area measurement. Bottom-right panel: Cardiac fibrotic area volume. (e) Representative cardiac function values obtained by echocardiography. All quantifications are derived from n = 10 mice/group and PCR experiments were performed with 2 replicates each. All quantitative data are presented as means ± SEM. ***p < 0.001 determined by 2-way ANOVA followed by Bonferroni post hoc test. Abbreviations: LDH: lactate dehydrogenase; ECHO: echocardiography.

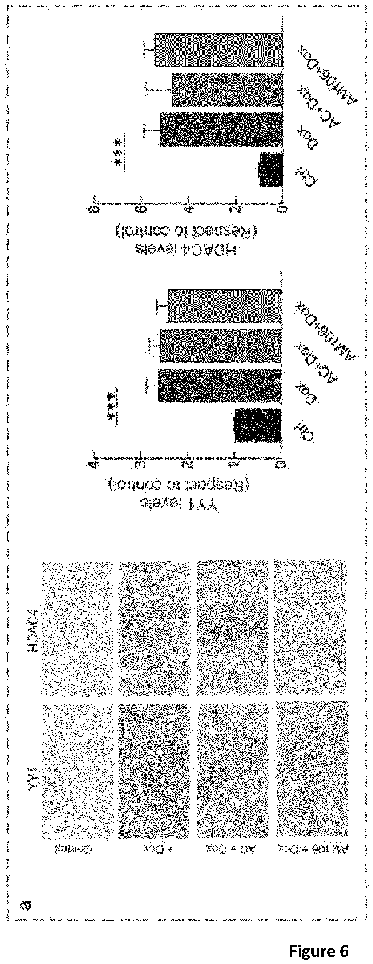

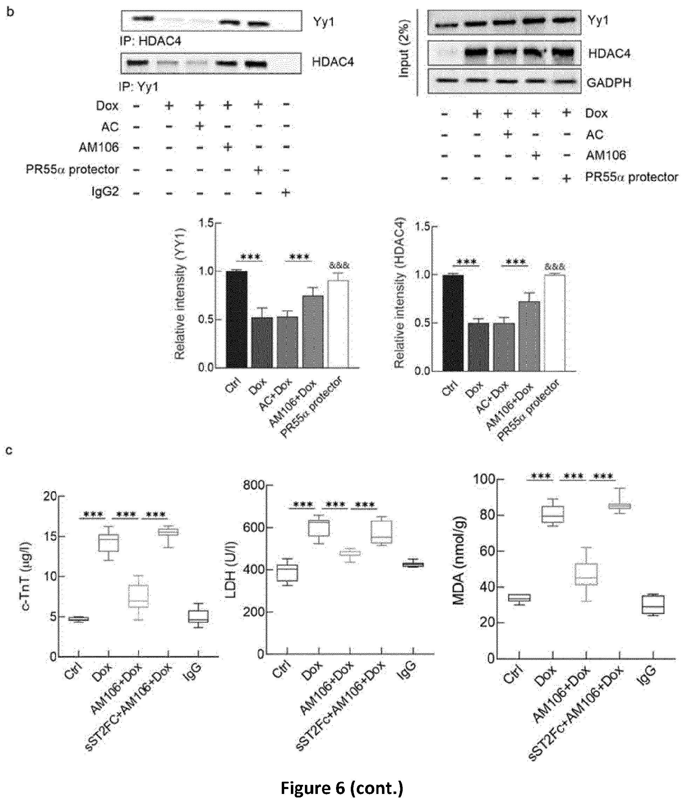

Fig 6. Anti-miR-106b therapy promotes nuclear interaction between HDAC4 and YY1. (a) Representative immunohistochemistry images and analysis; (b) Co-immunoprecipitation assays, representative western blots and analysis. (c) Levels of c-TnT, LDH activity and MDA levels. All quantifications are derived from n = 10 mice/group. All quantitative data are presented as means ± SEM. ***p < 0.001 determined by two-way ANOVA followed by Bonferroni post hoc test. Abbreviations previously described.

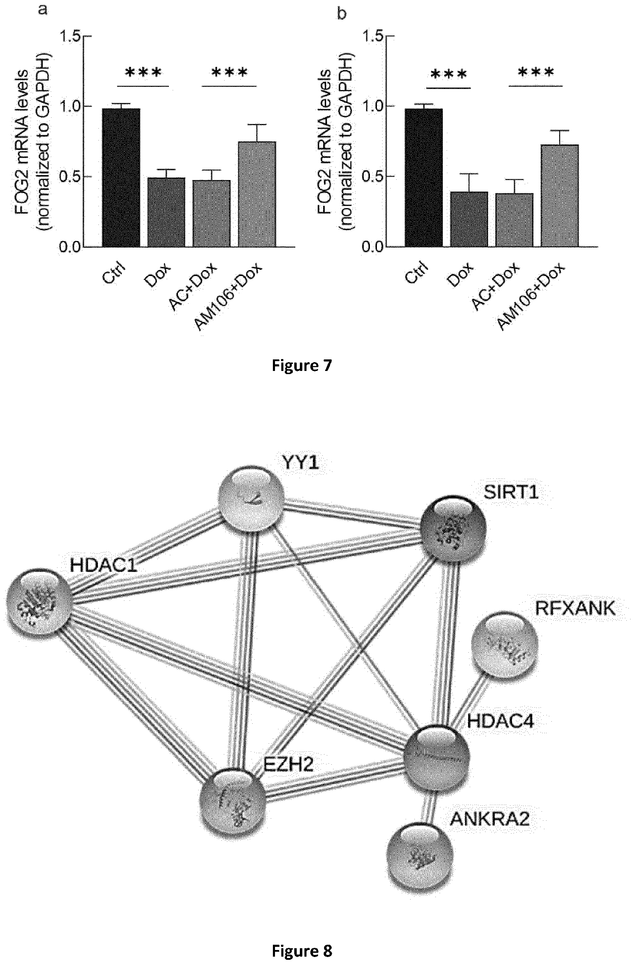

Fig 7. AntimiR106b therapy increases FOG2 mRNA levels both in hiPsCMs as well as HL-1 cell line in presence of 5 µM Dox for 15h. Quantitative real-time PCR analysis of FOG2 in hiPsCMs (panel a) or in HL-1 cell line (panel b) in presence of Dox; n=5 clones pergroups. All quantitative data are presented as means ± SEM. ***p < 0.001 determined by two-way ANOVA followed by Bonferroni post hoc test. Abbreviations previously described.
Fig 8. YY1 interacts with HDAC4 in human hearts. Data analysis obtained from STRING database (http://string-db.org/), allowed us to determine that YY1 interacts with HDAC4 in the murine heart with a score of 0.93.

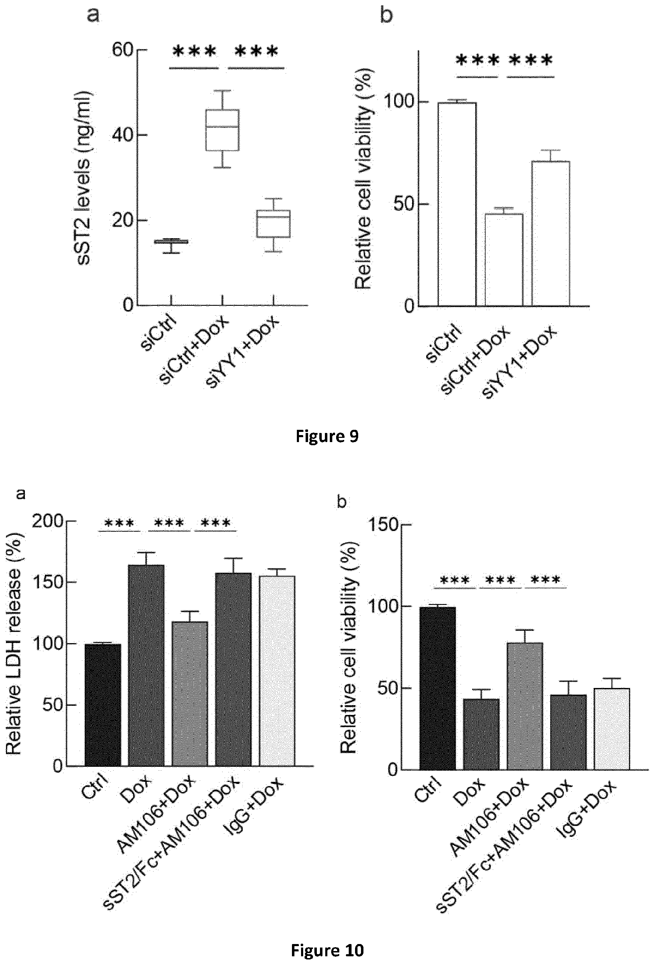

Fig 9. Silencing of YY1 was able to block preventive action of AM106 in hiPsCMs exposes to 5 µM Dox for 15h. p<0.001. (a) Measurement of sST2 levels in supernatants obtained from hiPsCMs exposed to 5 µM Dox for 15h under different experimental conditions. (b) Cellular viability of hiPsCMs exposed to 5 µM Dox for 15h under different experimental conditions. All quantitative data are presented as means ± SEM. ***p < 0.001 determined by two-way ANOVA followed by Bonferroni post hoc test. Abbreviations previously described
Fig 10. The addition of sST2 in parallel with AM106 treatment was able to block the preventive effect of AM106 in hiPsCMs treated with 5 µM Dox for 15h. (a) LDH levels measurement in supernatants obtained from hiPsCMs exposed to different experimental treatments. (b) Cellular viability of hiPsCMs exposed to different experimental treatments. All quantitative data are presented as means ± SEM. ***p < 0.001 determined by two-way ANOVA followed by Bonferroni post hoc test. Abbreviatures: LDH: lactate dehydrogenase; sST2/Fc: soluble ST2 isoform Fc fusion protein. Others abbreviatures as before.

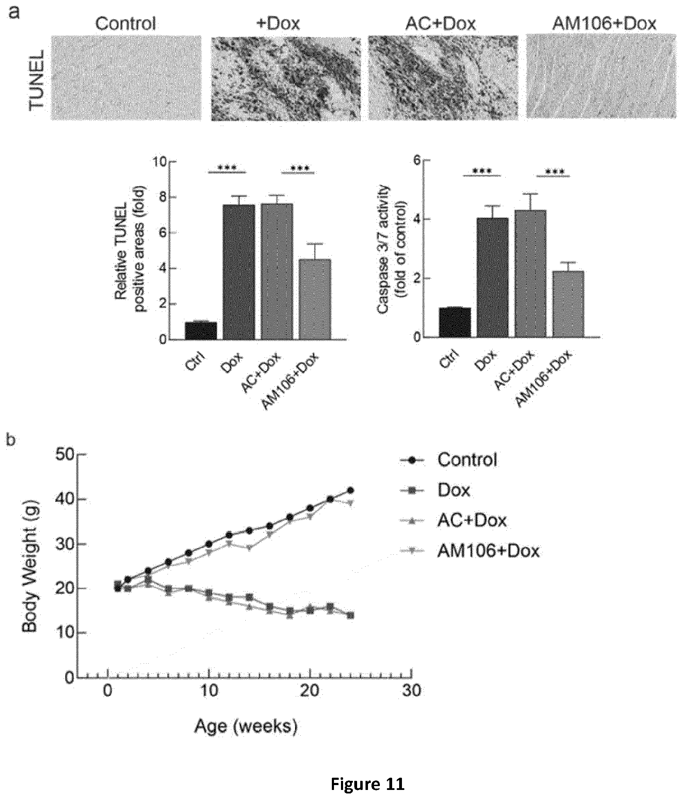

Fig 11. AM106 therapy prevented Dox-induced cellular death by apoptosis the whole-body cachexia, the loss of body mass and it was able to prevent the mortality associated with Dox treatment. (a) Representative microphotographs and analysis of TUNEL staining and caspases 3/7 activity measurements obtained from LV myocardium tissue, under different experimental procedures. (b) Body weight kinetic from mice under different treatments. (c) Overall Mortality Analysis from mice under different treatments. All quantitative data are presented as means ± SEM. ***p < 0.001 determined by two-way ANOVA followed by Bonferroni post hoc test. Abbreviations previously described

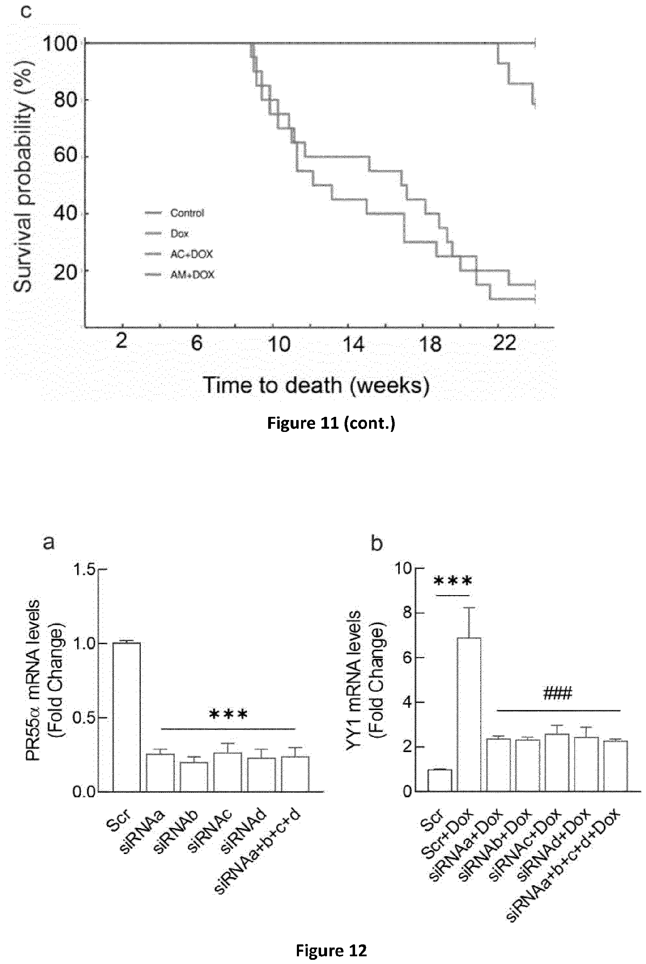

Fig 12. HiPsCMs were preincubated with scramble siRNA (scr), with a combination of four siRNAs to PR55α (siRNA PR55α_a+b+c+d), YY1 (siRNA YY1_a+b+c+d), or single siRNAs. The mRNA expression of PR55α (a) and YY1 (b) was analyzed by quantitative RT-PCR. ***p<0.001 vs control-Scr. ### p<0.001 vs Scr+Dox. Abbreviatures: Src: Scramble.

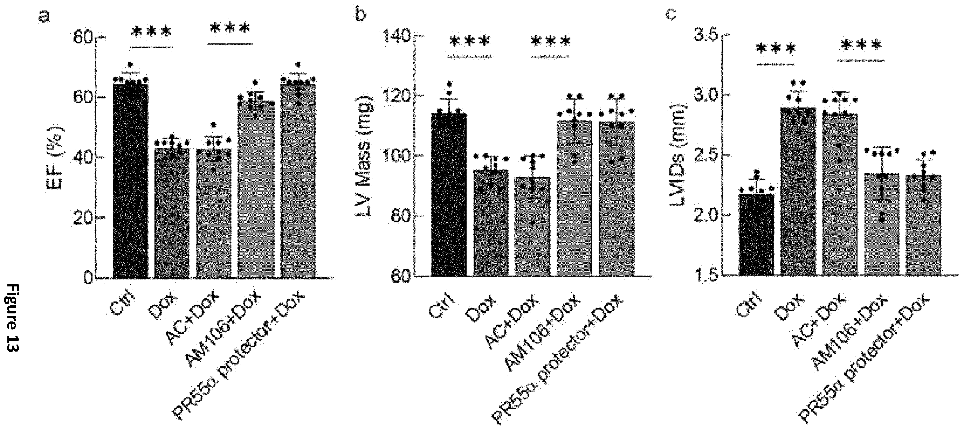

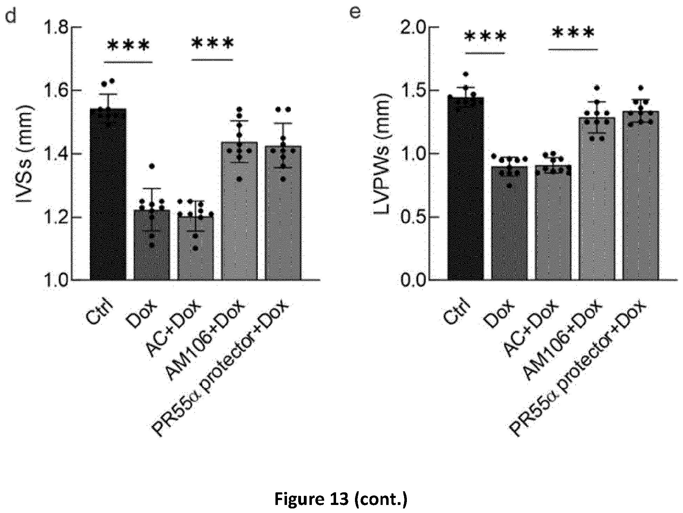

Fig 13. Mice pretreated with AM106 therapy improves their cardiac function in presence of Dox. Echocardiographic analyses of control hearts (n = 10), hearts with Dox (n = 10), AC-heart exposed to Dox (n=10), AM106 hearts exposed to Dox (n = 10), and PR55α protector hearts exposed to Dox (n=10) showing ejection fraction (a), left ventricular mass (b), left ventricular internal diameter systole (c), interventricular septum systole (d), and left ventricular posterior wall thickness systole (e). All quantitative data are presented as means ± SEM. ***p < 0.001 determined by two-way ANOVA followed by Bonferroni post hoc test. Abbreviations previously described.

### DESCRIPTION OF EMBODIMENTS

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity indicates that a number can vary between ± 20 % around its indicated value. Preferably "about" means ± 10 % around its value, more preferably "about" means ± 10, 8, 6, 5, 4, 3, 2 % around its value, or even "about" means ± 1 % around its value, in that order of preference.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of" does not exclude materials or steps that do not materially affect the basic and novel characteristics of the claim.

By "oligonucleotide" as referred herein is meant any short segment of DNA, RNA, or DNA/RNA, including both natural and synthetic nucleotides. As used in this invention, the term "oligonucleotide molecules" includes both oligonucleotides as such, as well as the "oligonucleotide analogues". "Oligonucleotide analogues" are the molecules derived therefrom that incorporate some chemical modification in at least one of the nucleotide units that form them, either in the phosphate group, the pentose or one of the nitrogenous bases; the modifications consisting in the addition of non-nucleotide groups at the 5' and/or 3' ends are also included as well as phosphorodiamidate morpholino oligomers, peptide nucleic acids (PNAs; mimics of DNA in which the deoxyribose phosphate backbone is replaced by a pseudo-peptide polymer to which the nucleobases are linked), and the like. By extension, for the purposes of this invention and as used herein, the terms "oligonucleotide molecule" and "oligonucleotide analogue" or "oligonucleotide analogue molecule" also include sponges of microRNAs or microRNA sponges, as it can be considered that the main constituent of the same are tandem repeats of oligonucleotides, characterized in that each of these oligonucleotides are in themselves or contain a binding site of a microRNA of interest. "Oligonucleotide sequence" is a term relating to the composition of an oligonucleotide.

"Percentage of sequence identity" for polynucleotides and polypeptides is determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by computerized implementations of known algorithms (BLAST in the resources of the National Center for Biotechnology Information, CLUSTAL in the resources of the European Bioinformatics Institute, GAP, BESTFIT, FASTA, and TFASTA in the Wisconsin Genetics Software Package, Genetics Computer Group (GCG), Science Dr., Madison, Wis.), or by inspection. Another indication that polynucleotide sequences are identical is if two molecules hybridize to each other under stringent conditions. Stringent conditions are sequence dependent and will be different in different circumstances. Generally, stringent conditions are selected to be about 5 DEG C. lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions for a Southern blot protocol involve washing at room temperature with a 5.times.SSC, 0.1% SDS wash.

As used herein, "antimiRs" refer to oligonucleotides, preferably oligoribonucleotides, that are complementary to a microRNA, preferably a mature microRNA, that is their target and they bind to with great affinity. Therefore, antimiRs refer to oligonucleotides, usually chemically modified with respect to the corresponding oligomer composed only of nucleotide units, and that are complementary to a target microRNA. In the particular case of the present invention, the antimiRs described herein are at least partially complementary to human miR-106b-5p as represented in SEQ ID NO 1.

As used herein, "microRNA sponges" are usually designed so that they inhibit microRNAs with a complementary heptameric or octameric fragment (seed region), such that a single sponge construct can be used to block a whole family of microRNAs sharing the same motif, although they may also contain the entire target sequence for a specific microRNA or only a miRNA-specific region, devoid of the seed region, to make it specific.

The expressions "pharmaceutically acceptable" or "pharmacologically acceptable" refer to molecular entities and compositions that do not produce any adverse, allergic or other reactions when administered to an animal or human being. As used herein, "pharmaceutically acceptable vehicle" includes solvents, buffers, solutions, dispersion media, coatings, antibacterial and antifungal agents, isotonic and absorption retarding agents and similar acceptable for use in formulation pharmaceuticals, such as pharmaceutical products suitable for administration to human beings.

"Preventing", "to prevent" or "prevention" or any other similar term, include without limitation, decreasing, reducing or ameliorating the risk of a symptom, disorder, condition, or disease, and protecting an animal from a symptom, disorder, condition, or disease. A prevention may be applied or administered prophylactically.

"Treating", "to treat" or "treatment" or any other similar term, include without limitation, restraining, slowing, stopping, reducing, ameliorating, or reversing the progression or severity of an existing symptom, clinical sign, disorder, condition, or disease. A treatment may be applied or administered therapeutically.

### DETAILED DESCRIPTION AND EMBODIMENTS OF THE INVENTION

In the present invention, we provide a therapy to treat and/or prevent the CTX developed in oncologic patients after receiving anthracycline-based chemotherapy. We provide the first evidence that links Dox-induced CTX with an increase in both miR-106b-5p levels as well as the cardiac sST2 isoform. Here, it is demonstrated that miR-106b-induced PR55α degradation leads to phosphorylated HDAC4 accumulation in the cytosol, activation of YY1 transcription factor and up-regulation of expression and release of the cardiac sST2 isoform. However, the combined use of Dox with an anti-miR-106b-5p was able to decrease cardiac sST2 expression, which was associated with a lower cardiac damage and, most importantly, a significative decrease in the mortality associated with the chemotherapeutic treatment.

It is well documented that Dox is able to exert dose-dependent CTX in oncologic patients. In fact, we found that mice exposed to Dox exhibited loss in body and heart weight (Figure 5); increased cardiac apoptosis and atrophy (**figure 11**); and, importantly, a decline in cardiac function (Figure 5e and **figure 13**), which leads to the development of HF. In this invention, we have focused our attention on miRNA-106b-5p (miR-106b), a miRNA that despite being related to the development and pathophysiology of different types of cancer and to be involved with oxidative damage and apoptotic cellular death, there are no studies, however, that have evaluated its implication in Dox-induced CTX. In this sense, we have observed both *in vitro* and *in vivo* an increase of myocardial expression and releases of miRNA-106b-5p (miR-106b) in response to Dox treatment. In this scenario, it was tempting to speculate that miR-106b could be a possible mediator of the negative consequences driven by Dox and therefore its functional blocking may have a cardioprotective effects. In order to demonstrate such hypothesis, we performed a systemic blocking of miR-106b and determined that such blocking was able not only to prevent the loss of muscle mass and cardiac atrophy associated with Dox treatment (Figures 5 and **figure 11b**), but also induced an improvement in myocardial functionality (Figure 5e and Figure 13) as well as a significative reduction in the mortality associated with chemotherapeutic treatment (**figure 11c**)**.**

Here, we further demonstrate that Dox-induced miR-106b-5p increase leads to the accumulation of the phosphorylated HDAC4 in the cytosol, losing its capacity of acting as a genetic YY1 co-repressor, which is associated with sST2 expression and release (Figures 4 and 9). Further, our data suggests a relationship between cytosolic HDAC4 accumulation to a loss of PP2A activity due to decreased expression of PR55α by miR-106b-5p. This functional role was confirmed using different experimental strategies. First, anti-miRNA-106b-5p (miR-106b) therapy prevented Dox-induced PR55α degradation, which lead to PP2A activation and a decrease in the phosphorylation state of HDAC4 on its activating site Ser246. These results were confirmed overexpressing miR-106b-5p levels or using specific sequences to block interaction site of miR-106b-5p on PR55α. Further, here we established the pathophysiological relationship both of YY1 as well as sST2 with Dox-induced CTX. Note that, endogenous silencing of YY1 in hiPsCMs exposed to Dox was able to prevent Dox-induced sST2 overexpression which lead to decreased CTX. In this setting, our data demonstrates the deleterious role of sST2 on Dox-induced CTX. Thus, the exogenous addition of sST2 both in hiPsCMs as well as mice treated with AM106b and exposed to Dox, was able to prevent the analysed cardioprotective effects related to the anti- miRNA-106b-5p (AM106b) (**Figure 6c** **and** **figure 10**).

In summary, it is herein demonstrated that an increase in Dox-induced miRNA-106b-5p (also referred herein as miR-106b) is able to down-regulate PR55α subunit, which leads to a decrease both in PP2A activity as well as an accumulation of the phosphorylated HDAC4 protein in cytosol. Anti-miRNA-106b-5p therapy is thus able to prevent Dox-induced PR55α degradation, facilitate the HDAC4 dephosphorylation by PP2A, and increase HDAC4-YY1 interaction, resulting in a decreased in sST2 expression and release by the LV myocardium. Further, and for first time, it has identified the role that sST2 isoform plays in Dox-induced CTX.

In view of these results, in **a first aspect**, the present invention relates to an oligonucleotide and/or oligonucleotide analogue molecule which is an antagonist of the human miRNA-106b-5p, preferably in a pharmaceutical form suitable for use in a method to treat or prevent one or more symptoms of the cardiotoxicity that is developed in oncologic patients after receiving or being exposed to anthracycline-based chemotherapy.

Since this invention focuses on reducing the activity of miRNA-106b-5p, it is its endogenous activity that will be diminished by the presence of its antagonists. In the context of the present invention, inhibitors, silencers or blockers are compounds that are capable of producing a decrease in the endogenous activity of said miRNA-106b-5p, and thus these three terms have been included under the denomination of "antagonist". While, strictly speaking, the term "silencing" could be interpreted as the absolute annulment of such activity, since the difference between such annulment or a non-absolute decrease in repressive activity may depend on the concentration of the compound used, it will be sufficient for a compound to result in a decrease in the activity of a microRNA to be considered an inhibitor, silencer, blocker or, in short, an antagonist thereof. In addition, taking into account the knowledge about the possibility of inhibiting microRNA function by targeting the mature microRNA, the precursor microRNA (pre-microRNA) or the primary microRNA (pri-microRNA), a compound could be considered a microRNA inhibitor, silencer, blocker or antagonist according to the present invention if it targets the mature microRNA, but also if it targets the precursor microRNA or the primary microRNA, provided that it is capable of producing a decrease in the endogenous activity of said microRNA. Therefore, as used herein, the four terms (inhibitors, silencers, blockers or antagonists) are used as synonyms in this specification.

To design the antimiR antagonists of the present invention, it is important to note that there should be sufficient complementarity with the endogenous molecules to which they must bind (in this case, the miRNA-106b-5p). In particular, the sequence of nucleotides of the miRNA-106b-5p must be taken into account for the design of the sequence of antimiRs and their microRNA binding site. The "microRNA binding site" is the nucleotide sequence comprised in the antimiR that is complementary or partially complementary to at least a portion of its target microRNA. In this case, the microRNA binding site of the antagonists defined herein are complementary or partially complementary to at least a portion of the miRNA-106b-5p as defined in SEQ ID NO 1. The sequence of the binding site can be a perfect match, meaning that it has perfect complementarity to the microRNA. Alternatively, the sequence can be partially complementary, meaning that one or more mismatches may occur when the microRNA is base paired to the binding site of the antimiR. Importantly, if the antimiR is partially complementary to the target microRNA (the miRNA-106b-5p) its binding site preferably contains perfect or near perfect complementarity (90%, 91% 92%, 93%, 94%, 95%, 96%, ,97%, 98%, 99% or preferably 100% complementary) to a region of the target microRNAs (the miRNA-106b-5p) that effectively prevents the miRNA (miRNA-106b-5p) from hybridizing with its normal cellular interaction partners.

### SEQ ID NO 1. miRNA-106b-5p (MIMAT0000680) 5'- UAAAGUGCUGACAGUGCAGAU -3'

In an embodiment, the oligonucleotide and/or oligonucleotide analogues molecules of the first aspect of the invention are inhibitors, blockers or antagonists of the types known as antimiRs and microRNA sponges. Preferably, the oligonucleotide and/or analogue thereof according to the first aspect or any of its embodiments is an antimiR.

In an embodiment, the oligonucleotide and/or analogue thereof which is an antimiR is at least 10, 11, 12, 13, 14, 15, 16, 17, 18, 19, 20, 21, 22, 23, 24, 25 nucleotides in length. In an embodiment, the oligonucleotide and/or analogue thereof is between 10-25 nucleotides in length, more preferably between 14-22 nucleotides in length. Preferably, the oligonucleotide molecule and/or analogue thereof is an antimiR whose sequence comprises, consists, or consists essentially of a fragment composed of a succession of at least 7, 8, 9, 10, 11, 12, 13, 14, 15, or 16 consecutive nitrogen bases of nucleotide or nucleotide analogue units that are identical in at least 60%, 70%, 80%, 85%, 90%, 91%, 92%, 93%, 94%, 95%, 96%, 97%, 98%, 99% or 99.5%, or 100% to the complementary sequence of a region as present in miRNA-106b-5p of SEQ ID NO 1. More preferably, the full-length sequence of the nitrogen bases of the nucleotide or nucleotide analogue units comprised in the oligonucleotide molecule and/or analogue thereof is identical in at least 60%, 70%, 80%, 85%, 90%, 91%, 92%, 93%, 94%, 95%, 96%, 97%, 98%, 99% or 99.5%, or 100% to the full length complementary sequence of miRNA-106b-5p of SEQ ID NO 1.

In an embodiment, the antagonist is an antimiR whose sequence comprises a first fragment and a second fragment, wherein the first fragment is composed of a succession of at least 5-8 nucleotide or nucleotide analogue units wherein the sequence of the nitrogenous bases of said first fragment is identical in at least 90%, 91%, 92%, 93%, 94%, 95%, 96%, 97%, 98%, 99% or 100% to the complementary sequence of a region of the target microRNAs (the miRNA-106b-5p) that effectively prevents the miRNA (miRNA-106b-5p) from hybridizing with its normal cellular interaction partners, and wherein the second fragment is adjacent to the first fragment (i.e., it is located upstream and/or downstream of the first fragment) and it is composed of a succession of at least 7, 8, 9, 10, 11, 12, 13, 14, 15, or 16 consecutive nitrogen bases of nucleotide or nucleotide analogue units that are identical in at least 60%, 70%, 80%, 85%, 90%, 91%, 92%, 93%, 94%, 95%, 96%, 97%, 98%, 99% or 99.5%, or 100% to the complementary sequence of said region present in SEQ ID NOs: 1.

As used in this specification, it is understood that two chains of nucleotide molecules are 100% complementary when the nucleotide or nucleotide analogue sequence of one of them, read in the 5'-3' sense, is the sequence of nucleotides or nucleotide analogues that present the nitrogenous bases which pair with the nitrogenous bases of nucleotides or nucleotide analogues of the other sequence, read in the 3'-5' sense. That is to say, the sequence 5'-UAGC-3' would be complementary to the sequences 3'-AUCG-5' and 3'-ATCG-5' which would be, respectively, sequences 5'-GCUA-3' and 5'-GCTA-3' read in the 5'-3' sense. In an embodiment, it is preferred that the antagonist molecule comprises in its sequence a fragment that is identical to the complementary sequence to that of the seed region of the microRNA to be antagonized, at least with regard to the complementarity of the nitrogenous bases. And often, especially in the case of antimiRs, modifications are incorporated to the corresponding nucleotide units, which mainly affect the ribose moiety and/or phosphate, modifications that are difficult to depict in the usual representations of nucleotide sequences, in which the nucleotide present in a given position is identified by the abbreviation of the nitrogenous base that is part of it. Therefore, in the present invention, there are compared molecules of microRNA antagonists that refer to the percentage of identity between the sequences of the nitrogen bases of the nucleotide or nucleotide analogue units present in these units, as this is what indicates whether two molecules or sequence fragments are designed from the same original basic nucleotide sequence, although different chemical modifications may have been included in the nucleotides in each case.

Also comprised within the concept of oligonucleotide and/or oligonucleotide analogue molecule useful for the purpose of the present invention and comprised within its scope are those microRNA inhibitors, blockers or antagonists that act on pri-microRNAs or pre-microRNAs, usually altering microRNAs biogenesis and having a negative effect on microRNAs activity, mainly due to a decrease of the active available microRNA. In animal cells, immature pri-miRNAs are processed into pre-miRNAs by the Microprocessor complex in the nucleus, and are then transported into the cytoplasm to undergo further processing into mature miRNAs. It thus must be understood that targeting the pri-microRNA and/or the pre- microRNA of miRNA-106b-5p and altering their biogenesis so that the levels of said microRNAs are decreased should also result in a decrease of their activity. Therefore, for the purpose of the present invention, an antagonist of miRNA-106b-5p must be understood to comprise not only those molecules capable of acting the mature forms, but also those molecules capable of acting on the pre-microRNA or the pre- RNA and decreasing the levels of the mature forms of miRNA-106b-5p. In order to design them, it must be taken into account that:
- The primary microRNA (pri-microRNA) of miRNA-106b-5p is
   HGNC Reference Sequence (SEQ ID NO 2): 
5´-
CUUUAAAUAAAAUAUAUGUUUGCCAUGUCAUGCGCUCAGAGGAGGGUCUGGAUCCUCUCUU
AAUGACAGACGGAUUUGUCUUACAAAGGCCAUUUUCCCGAAAAAAAUAGGAAAUAACUUGGAG
GAUACUGGUGGGGGCUAUACGUUUGGUGAAAUCCAUCAGUGAUAUUCAAGCCAAGCAAAUAAU
CACCCACUAAUUUGUGUUUCCACUUAAAAUUCUUGGAGGGGACCAUGUUAAAUGGGGAGCCC
 
UGGUACAGAAAACAACAUGGGCUCAUAUUCACUCCCUUCCCCAUCUCCCUCUUCUGCUUCCCC
AUGAACCUUCCCCCACAGGCGUUACAUAGCCAUGUGCCGCGAGAAGCAGCCCAUGGUGCCAGA
GUCUCUGGCUGACUACAUCACAGCAGCAUACGUGGAGAUGAGGCGAGAGGCUUGGGCUAGUAA
GGAUGCCACCUAUACUUCUGCCCGGACCCUGCUGGCUAUCCUGCGCCUUUCCACUGCUCUGGU
AAGUGCCCAAAUUGCUGGAGGGCCAUCUGUUUGACCCUUAAAGGGUAGCUCCUUACCGUGC
UCUCAUUGCCGCCUCCCCACCUCCCGCUCCAGCCCUGCCGGGGCUAAAGUGCUGACAGUGCAG
AUAGUGGUCCUCUCCGUGCUACCGCACUGUGGGUACUUGCUGCUCCAGCAGGGCACGCACAGC
GUCCGUGGAGGGAAAGGCCUUUUCCCCACUUCUUAACCUUCACUGAGAGGGUGGUUGGGGUCU
GUUUCACUCCAUGUGUCCUAGAUCCUGUGCUACAGACCUUCCUUUCUGUCCUCCCGUCUUGGA
CCUCAGUCCUGGGGGCUCCAAAGUGCUGUUCGUGCAGGUAGUGUGAUUACCCAACCUACUGCU
GAGCUAGCACUUCCCGAGCCCCCGGGACACGUUCUCUCUGCCAAUUGUCUUCUUGGCUGAGCU
CCCCAAGCUCCAUCUGUCAUGCUGGGGAGCCCAGUGGCGUUCAAAGGGUCUGGUCUCCCUCA
CAGGACAGCUGAACUCCGGGACUGGCCAGUGUUGAGAGGCGGAGACUUGGGCAAUUGCUGGAC
GCUGCCCUGGGCAUUGCACUUGUCUCGGUCUGACAGUGCCGGCCCAACACUGCGGAUGCUGGG
GGGAGGGGGGAUUCCACUCCUGUUUUGUGAGUAGGCGACCCAUGGGCUGCCCAGCCUUAAAGC
CAGAACAAGGGUGUCCCCUGACCUCGUUCCACUGCCCUCCUCCCGUUCCCAUCUUUCCCCCCU
ACCUUCCCCUUAGGCACGUCUG-3´
 Preferably, in order to avoid undesired secondary effects, if it is desired to have an antagonist targeting this pri-microRNA, it would be preferable to select it so that other microRNAs of the same cluster are not affected. For that same reason, it is preferred that an antagonist of miRNA-106b-5p is designed so that it is an antagonist of the mature microRNA and/or of the pre-microRNA.
- The microRNA precursor (pre-microRNA) of miRNA-106b-5p corresponds to (SEQ ID No 4)

5'-
CCUGCCGGGGCUAAAGUGCUGACAGUGCAGAUAGUGGUCCUCUCCGUGCUACCGCACUGUGGG
UACUUGCUGCUCCAGCAGG -3'

As this is a longer sequence than that of miRNA-106b-5p, it is possible to design antagonist specific to the pre-microRNA.

On a further note and as shown in the examples below, the authors of the present invention developed and optimized an antimiR against miRNA-106b-5p. The specific sequence of this antimiR comprises SEQ ID NOs: 3 (AM106, YCI0202832) (5'-CACTGTCAGCACTTT-3' (SEQ ID NO 3)).

Thus, in an embodiment, the oligonucleotide molecule and/or analogue thereof is an antagonist of the human miRNA-106b-5p and it comprises, consists, or consists essentially of a fragment composed of a succession of at least 7, 8, 9, 10, 11, 12, 13, 14, or 15 consecutive nitrogen bases of nucleotide or nucleotide analogue units that are identical in at least 50%, 60%, 70%, 80%, 85%, 90%, 91%, 92%, 93%, 94%, 95%, 96%, 97%, 98%, 99% or 100% to the sequence of said region present in SEQ ID NO: 3 (**CACTGTCAGCACTTT**). Preferably, the full-length sequence of the nitrogen bases of the nucleotide or nucleotide analogue units comprised in the oligonucleotide molecule and/or analogue thereof is identical in at least 60%, 70%, 80%, 85%, 90%, 91%, 92%, 93%, 94%, 95%, 96%, 97%, 98%, 99% or 99.5%, or 100% to the full length sequence of the nitrogenous bases of the oligonucleotide to SEQ ID NO: 3.

It is noted that in the oligonucleotide molecule and/or analogue thereof according to the present invention, each uracil and thymine base within the full length of the oligonucleotide molecule and/or analogue, preferably each uracil and thymine base in the seed region, can be optionally replaced, respectively, by a thymine o uracil base. This applies for all the oligonucleotides disclosed herein.

The antimiRs of the present invention can be further optimized in order to improve their *in vivo* stability and efficacy. To do so, several modifications in their chemical architecture have been described (for a review, see Mckenzie et al., Recent progress in non-native nucleic acid modifications). These modifications can be made in the pentose (in the preferred embodiment in which the oligonucleotide is an oligoribonucleotide, the modification would be in the ribose), in the internucleotide linkage, or in the nucleobase, or in a combination thereof. When the oligonucleotides or oligoribonucleotides of the present invention are chemically modified, they are considered in the context of the present invention as oligonucleotide analogues or oligoribonucleotide analogues, respectively. In an embodiment, the antimiR is an oligonucleotide analogue and it comprises at least six, seven, eight, nine, then, eleven, twelve, thirteen, fourteen, or fifteen chemical modifications along the whole molecule. In an embodiment, the antimiR is an oligoribonucleotide analogue that comprises all its nucleotides chemically modified.

Modifications in the internucleotide linkage: It is considered included in the possible modifications that give rise to the oligonucleotides analogues of the present invention are the modifications that give rise to phosphorothioate bonds, which are modifications that affect phosphate groups that are part of the "skeleton" of the nucleotide chain, giving rise to the introduction of a sulphur atom in substitution of an oxygen atom of the phosphate group that is not acting as a bridge between nucleotides; these modifications cause the bonds between nucleotides to be resistant to degradation by nucleases, so they are commonly inserted between the last 3-5 nucleotides at the 5' or 3' ends of oligonucleotides to inhibit degradation by exonucleases, increasing their stability.

Modifications in the pentose, preferably in the ribose: The most widely used sugar modifications are those that are located in the OH group at the 2' position. Among them, the most important ones in the context of the present invention are 2'fluoro (2'F: introduction of a fluorine atome at the ribose 2' position), 2'M-O-methoxyethyl (MOE), or 2'O-methyl (OMe) modifications. Thus, in an embodiment, the antimiR according to the present invention is chemically modified to comprise at least one pentose with one of the following modifications: 2'fluoro ((2'F: introduction of a fluorine atome at the ribose 2' position), 2'M-O-methoxyethyl (MOE), and/or 2'O-methyl (OMe).

Another modification that can be performed in the antimiRs of the present invention is the formation of a bridge between the 2' oxygen and the 4' carbon and locks the ribose in the 3' endo conformation, which leads to a modification called *locked nucleic acids,* or LNA. The introduction of LNAs modifications highly increases the stability of the molecules, making them significatively more resistant to degradation, which especially happens when said modifications are placed in the ends of the molecule. In an embodiment, the first nucleotide starting from the 3' region comprises a LNA modification. In another embodiment, the each of the two first oligonucleotides starting from the 5' region comprise a LNA modification.

More modifications include BNAs ("Bridged nucleic acid')', PMOs (nucleic acids where ribose has been substituted by a carbonylchloride group), or PNAs ("Peptide Nucleic Acid': peptide nucleic acid in which the ribose-phosphate group is replaced by an amino acid moiety, so that the skeleton of the nucleotide analogue is a structure of repeat units of N-(2-aminoethyl)-glycine linked by peptide bonds).

Modifications in the nucleobase: Because of its frequent use within the group of antimiRs, also included among the chemical modifications that give rise to the oligonucleotides, preferably oligoribonucleodide analogues of the invention, is the 5' methylation of the nitrogenous base cytosine (C), which decreases the detection of the oligonucleotide analogue by the immune system. Thus, in an embodiment, at least one, two, three, four, five, or more than five of the nucleotides comprised in the oligonucleotide and/or oligonucleotide analogue molecule according to the first aspect or any of its embodiments comprises a methylated cytosine.

As can be deduced from the definition of "oligonucleotide molecules" and that of "oligonucleotide analogues", also included within the definition of oligonucleotide analogues are hybrid molecules, in which some units present modifications and others do not, as well as hybrids between analogues of nucleic acids and peptides or, even, hybrid molecules in which some of the nucleotide units are nucleotides (or analogues thereof) and others are deoxynucleotides (nucleotides in which the sugar is deoxyribose), as well as analogues of the latter, i.e. RNA-DNA hybrids and analogues thereof. Other chemical modifications are possible and known, which are also comprised within the possible modifications that give rise to oligonucleotide analogues.

With regard to the possible chemical modifications included in the oligonucleotide and/or oligonucleotide analogue molecule, the term will be applied especially in the case of one or more of the usual modifications known to those skilled in the art of molecular biology, in terms of basic research and, in particular, in the search for therapeutic applications of these molecules. Information on such modifications can be found in the general common knowledge.

Additionally, also comprised within the present invention are compounds such as oligonucleotide molecules and/or analogues thereof in the form of a prodrug, i.e., in a form or nature that is not fully active but that will be converted or metabolized within the body upon administration to give rise to the fully pharmacologically active oligonucleotides molecules and/or analogues thereof described herein.

In a **second aspect**, the present invention relates to a composition, preferably a pharmaceutical composition, comprising at least an oligonucleotide as defined in the first aspect or any of its embodiments, or a mixture of two or more of them, optionally further comprising a carrier and/or one or more pharmaceutically acceptable excipients. In one possible embodiment, the pharmaceutical composition comprises an effective dose of an inhibitor or antagonist of the human miRNA-106b-5p. Preferably, the inhibitor/antagonist of the human miRNA-106b-5p present is the antimiR type inhibitor used in the examples of this invention (represented by SEQ ID NO 3). More preferably, the inhibitor(s)/antagonist(s) present will be present at a concentration that allows the administration of a therapeutically effective dose.

An "effective dose" or "therapeutically effective dose" is a sufficient amount to achieve a beneficial or desired clinical outcome. The precise determination of what would be considered an effective dose in humans can be based on individual factors for each patient, including size, age, and the nature of the inhibitor or antagonist (for example, if it is an expression construct, an antimiR or oligonucleotide analogue, etc). Nonetheless, the dosages can be easily determined by ordinary experts skilled in the art based on this description and the knowledge in the art.

For its clinical application, the compositions according to the uses of this invention, will then be considered pharmaceutical compositions of this invention, and they can be prepared in an appropriate form for the desired application. It may be necessary or convenient to administer multiple doses to the subject during a particular treatment period, administering doses daily, weekly, monthly, every two months, every three months or every six months. It is noted that such treatment period can be prior to, simultaneous to or subsequent to the anthracycline-based chemotherapy. Preferably, the treatment is administered to an oncologic patient in need thereof prior to the anthracycline-based chemotherapy. Also preferably, the treatment is administered to an oncologic patient in need thereof simultaneously or subsequently to the anthracycline-based chemotherapy

Colloidal dispersion systems, such as macromolecule complexes, nanocapsules, micro-spheres, pearls and lipid-based systems that include oil-in-water emulsions, micelles, mixed micelles, other oligonucleotide-based delivery vehicles, and liposomes, can be used as administration vehicles of the inhibitors/antagonists of this invention, with which the pharmaceutical composition of the invention is formed. Another possibility is to prepare the pharmaceutical compositions of the invention using appropriate salts and buffers to make the administration vehicles stable and to assist in the capture by the target cells. The compositions of this invention can be aqueous compositions that comprise an effective amount of the administration vehicle and which comprise either the oligonucleotide molecules of the invention, independently or forming liposomes or other complexes, or expression vectors thereof, dissolved or dispersed in a pharmaceutically acceptable carrier or aqueous medium.

Additional active ingredients may also be incorporated into the compositions, provided that they do not inactivate the molecules of this invention or their expression vectors.

The solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary storage and use conditions, these preparations generally contain a preservative to prevent the growth of microorganisms. The oligonucleotides can also be prepared in a solution of phosphate buffered saline and sodium chloride.

The compositions of this invention can usually be formulated in a neutral or salt form. Pharmaceutically acceptable salts include, for example, acid addition salts (formed with free amino groups of the protein) derived from inorganic acids (e.g., hydrochloric or phosphoric acids), or organic acids (e.g. acetic, oxalic, tartaric, mandelic acids), and the like. Salts formed with free carboxyl groups of the protein can also be derived from inorganic bases (for example, sodium, potassium, ammonium, calcium, or ferric hydroxides) or organic bases (e.g. isopropylamine, trimethylamine, histidine, procaine, and the like).

In a **third aspect**, the present invention provides an oligonucleotide as defined in the first aspect or any of its embodiment, or a composition, preferably a pharmaceutical composition, as defined in the second aspect or any of its embodiments for use in a method to treat or prevent one or more symptoms of the cardiotoxicity that is developed in oncologic patients after receiving anthracycline-based chemotherapy. It is noted that such risk of cardiotoxicity is the most serious drawback to the clinical usefulness of anthracycline antineoplastic antibiotics, which can be preferably selected from the list consisting of doxorubicin (Dox) (adriamycin), daunorubicin or epirubicin. It is again further noted that such treatment can be administered prior to or simultaneous to the anthracycline-based chemotherapy. Preferably, the treatment is administered to an oncologic patient in need thereof prior to the anthracycline-based chemotherapy. Also preferably, the treatment is administered to an oncologic patient in need thereof simultaneously to the anthracycline-based chemotherapy

In a preferred embodiment of the third aspect of the invention, the method is to treat or prevent the loss of muscle mass and cardiac atrophy that is developed in the oncologic patients after receiving anthracycline-based chemotherapy. In another preferred embodiment of the third aspect of the invention, the method prevents, reduces or mitigates the myocardial dysfunction in oncologic patients after receiving anthracycline-based chemotherapy.

In another preferred embodiment of the third aspect of the inventon or of any of its preferred embodiments, the anthracycline-based chemotherapy is selected from the list consisting of doxorubicin (Dox) (adriamycin), daunorubicin or epirubicin, preferably the anthracycline-based chemotherapy is doxorubicin (Dox).

In an embodiment of the third aspect, the treatment is a palliative treatment of one or more symptoms of the cardiotoxicity that is developed in oncologic patients after receiving anthracycline-based chemotherapy.

In an embodiment of the third aspect, the subject in need thereof is a mammal, preferably a human being, preferably an oncologic patient, which shall or is treated with anthracycline-based chemotherapy.

Given the stability of the antimiRs, direct administration to mammals, preferably human beings, can be considered, for example via subcutaneous or systemic routes, preferably intravenously or intrathecal, for example dissolved or suspended in a pharmaceutically acceptable carrier, such as water or an aqueous solution such as saline or phosphate buffer, or intraarticular delivery. The composition in which they are administered may contain pharmaceutically acceptable excipients.

The active compositions of this invention can be administered by any of the common routes, provided that the target tissue is available through that route. This includes oral, nasal, intrathecal, or buccal routes and, preferably, administration may be via an intradermal, subcutaneous, intramuscular, intraperitoneal, or intravenous route. As previously commented, it is common for compositions comprising antimiRs to be formulated for intravenous or subcutaneous administration.

After formulation, the solutions are preferably administered in a form that is compatible with the dosage formulation and in such a quantity that it is therapeutically effective. Formulations can be easily administered in a variety of dosage forms such as injectable solutions, drug release capsules, and the like.

The following examples merely illustrate the present invention.

### EXAMPLES

### Abbreviations

AC: AntimiR-control
AM: antimiRNA
CTX: Cardiotoxicity
Dox. Doxorubicin
HDAC4: Histone deacetylase 4
HF: Heart failure
NC: Negative control
PR55α: Protein Phosphatase 2 Regulatory Subunit B Isoform A
ROS. Reactive oxygen species
sST2: Soluble suppression of tumorigenesis-2
YY1: Yin yang-1 transcription factor

### Materials and methods

### Human Induced Pluripotent Stem Cell-Derived Cardiomyocytes.

Human induced pluripotent stem cells (hiPCs) were obtained from Phenocell^{®}, (Catalog No. PCi_CAU) and cultured according to manufacturer's instructions. Briefly, hiPCs on a suitable matrix to allow attachment of cell aggregates (Matrigel^{®}), were grown to 80% confluence using mTeSR^{™} plus medium. At passage 20, we started reprogramming procedure using a standardize protocol in our laboratory. Thus, culture medium was removed and changed to RPMI-1640 with B27 minus insulin supplement (basal differentiation supplement) with 4 µM CHIR99021 (day 0). On day 3, medium was changed to basal differentiation supplement and 3 µM IWR-1. On day 5, medium was refreshed with basal differentiation supplement. Medium was changed on day 8 to RPMI 1640 with B27 supplement (cited above as RMPI/B27). On day 11, medium was changed to RPMI 1640 without glucose with B27 minus insulin. On day 14, medium was changed to RPMI/B27; procedure that was repeated every 72 h. Beating clusters were observed after 7-14 days. A standardizing gene expression profiling (RT-qPCR) both hiPs as well as hiPsCMs were carried in each human isolated clone, before starting assays. Specific primer sequences were detailed in Table 1. The cardiotoxic treatment was carried out by exposing the cell culture to 5 µM doxorubicin for different time period; unless otherwise mentioned. All the in vitro assays were performed as three biological replicates, with technical replicates each time.

### Transfection procedure.

The hsa-miR-106b-5p sequence of nucleotides was obtained from www.MiRbase.org. Human miR-106b (accession No: MIMAT0000680) and nucleotide sequence of 5'-UAAAGUGCUGACAGUGCAGAU-3' are homologous between mice and man. In order to express or block the specific miR-106b function, synthetic pre-miR-106b or antimiR-106 oligonucleotides were transfected into hiPsCMs cells, respectively. Lipofectamine RNAiMAX transfection reagent (ThermoFisher) was used according to the manufacturer's instructions. The miRCURY LNA microRNA Inhibitor^{™} (AM106, YCI0202832) (5'-CACTGTCAGCACTTT-3'), the MiRCURY LNA miRNA Mimic (106b mimic, YM00471973) (5'-UAAAGUGCUGACAGUGCAGAU-3'), together with their respective controls (AC, YCI0200428; 5'-ACGTCTATACGCCCA-3') (NC, YM00479903; 5'-GAUGGCAUUCGAUCAGUUCUA-3') were purchased from Qiagen (Sequences are showed without the LNA pattern). Two day before transfection, hiPsCMs cells were seeded in 6-well plates with 2 ml of STEMdiff^{™} Cardiomyocyte Support Medium (STEMCELL^{™} Technologies) to a density of 60%. Stock transfection mixes were made according to manufacturer's instructions. Briefly, 9 µl of Lipofectamine reagent was diluted in 141 µl of Opti-MEM I Medium (Invitrogen) and incubated 5 min at RT. In another tube, pre-miRNA, antimiRNA or the specific controls oligonucleotides were diluted with Opti-MEM to a final concentration of 50 nM. Both mixes were incubated together for 30 min at RT to allow complex formation between miRNA and lipids. To transfect cells, the STEMdiff^{™} Cardiomyocyte Support Medium was removed and replaced with 1,75 ml fresh Medium and 250 µl of the appropriate transfection mix. Cells were incubated at 37 ºC for 48 days, after which hiPsCMs were treated with 5 mM Dox for 15 h. Transfection efficiency was tested in parallel using both hiPsCMs as well as a mouse cell line HL-1 (**figure 7**). The experimental procedure to silence endogenous PR55α or YY1 mRNA levels in hiPsCMs was similar to described above. Here, naive cells were washed and transfected with four interference RNA sequences specifically designed to human PR55α (5' GAAAUUACAGACAGGAGUU-3', 5'-UAUCAAGCCUGCCAAUAUG-3', 5' UAUGAUGACUAGAGACUAU-3', and 5'-GCAGAUGAUUUGCGGAUUA-3') or to human YY1 mRNA sequence (A-011796-16-0005, A-011796-17-0005, A-011796-18-0005 y A-011796-16-0005) or with a non-targeting Accell siRNA sequences (siCtrl) (D-001810-10-05) siRNA. The transfection concentration was 25 nM, and the efficiency was determined and is showed in **figure 7****.** To block the specific miR-106b-5p/PR55α interaction a miRCURY LNA miRNA Power Target Site Blockers (Qiagen) (PR55α protector) was used (50 nM final concentration) (Seed sequence to be masked: 5'-CACUUU-3'). When indicated, sST2-Fc or IgG (100 nM in both cases) were added 24 h before AM106 therapy.

### Assay for cell viability.

After seeding 5 × 10³ cells in each 96-well plate, they were incubated in 5% CO2 at 37 °C for 24h. After cited treatment, MTT solution (1 mg/ml) was added to each well and an incubation was performed for 3 h at 37 °C. Next, the medium containing MTT was removed and 100 µL of DMSO was added to each well and pipetted several times to form a completely uniform suspension. The solution was then shaken for 10 min at 37 °C until formazan crystals were completely dissolved. The obtained solution was added to a microtube and centrifuged in 1500 RPM for 5 min to eliminate any impurities. Absorbance of the samples was read at 570 nm and the background value at 690 nm was subtracted. The intrinsic absorbance value of doxorubicin was subtracted when necessary. The index of cell survival at each time point corresponds to the percentage with respect to control.

### Caspase assay.

Caspase assay was performed as per the manufacturer's instructions with the Caspase-Glo 3/7 kit (Promega). In this setting, hiPsCMs were treated with 5 µM Dox for 15 h. Next, hiPsCMs and caspase assay reagent were left at RT for 30 min. The next step was adding an equal amount of caspase assay reagent and further incubated for 30 min at RT. Luminescence was measured using a microplate reader CLARIOstar Plus (BMG Labtech).

### PP2A phosphatase assay.

To measure PP2A phosphatase activity, we used a PP2A Immunoprecipitation Phosphatase Assay Kit (Millipore Sigma, catalog number 17-313). In brief, cells were lysed in 20 mM imidazole HCI, 2 mM EDTA, 2 mM EGTA, pH 7.0 with 10 µg/mL each of aprotinin, leupeptin, pepstatin, 1 mM benzamidine, and 1 mM PMSF. Two milligrams of the lysates were then immunoprecipitated with 2 µg of anti-STRN4 antibody (Abcam, ab177155) and 40 µl of protein-A-agarose beads at 4°C overnight. Beads were washed three times with lysis buffer followed by the Ser/Thr assay buffer. Phosphatase reactions were then performed in Ser/Thr assay buffer with a final concentration of 750 µM of MAP4K4 phosphopeptides: S771/ S775 (A-A-S-pS-L-N-L-pS-N-G-E-T-E-S-V-K), S876 (L-T-A-N-E-T Q-pS-A-S-S-T-L-Q-K) or S1251 (V-F-F-A-pS-V-R-S) for 10 min at 30°C. To provide evidence that the immunoprecipitated phosphatase activity is PP2A, we treated parallel immunoprecipitates with 5 nM of okadaic acid (Cell Signaling, #5934). Dephosphorylation of the phosphopeptide was measured through malachite green phosphate detection at 650 nm.

### Animals and ethics statement.

12-18-week-old C57BI/6J male mice (weighing 25-30 g) were purchased from the ENVIGO Laboratory. Mice were housed in specific pathogen free environment with a relative humidity of 50 ± 5% at 23 ± 2 °C with 12 h light and dark cycles. Mice had free access to food and water. The study protocol was examined and approved by the Institutional Ethic and Animal Experimentation Committee of the University of Murcia, according to Spanish Government Guidelines and European Community Guidelines for animal care (authorized number A13150105). All methods were carried out in accordance with relevant guidelines and regulations.

### Doxorubicin-induced cardiotoxicity animal model and treatments.

In the present study, C57BL6 mice were given Dox at a dose of 5 mg/kg intraperitoneally weekly for 5 consecutive weeks (cumulative dose: 25 mg/kg) to achieve CTX. Animals were sacrificed after eight weeks.

### LV structure and function.

A transthoracic echocardiographic examination was performed in all mice by a blinded trained investigator (LAX, A) before Dox treatment (baseline) and 8 weeks post-Dox administration. A Vevo 3100 high-frequency ultrasound imaging system (VISUALSONICS, Inc, Toronto, Canada) with a 30-MHz central frequency transducer was used with an integrated rail system III for imaging acquisition. The end-systolic (LVESV), diastolic left ventricular (LVEDV) volumes and ejection fraction (EF) were computed using the modified Simpson's method from the left parasternal long-axis view. Left ventricular (LV) diastolic and end-systolic dimensions were also obtained from parasternal short axis M-mode.

### In vivo delivery of LNA-antimiR oligonucleotides and experimental design.

To achieve a systemic loss of function of miR-106b, AM106 (20 mg/kg, i.v.) was injected to the mice seven days before the first Dox injection (5 mg/kg, i.p.). Next, mice were injected for five consecutive weeks, and simultaneously, with 5 mg/kg Dox and 20 mg/kg AM106 and eight-week later sacrificed (Figure 5a). Control animals and mice treated with Dox were then randomized into control or treated groups. Animals were randomly divided into four experimental groups: (1) control mice receiving saline for 8 weeks (wks); (2) mice treated with Dox receiving saline for 8 wks; mice treated with Dox receiving antimiR-Ctrl for 8 wks; and (4) mice treated with Dox receiving AM106 for 8 wks. To investigate the effect of sST2-Fc mice received i.p. (caudal vein) injections of recombinant sST2-Fc at doses of 100 µg/mouse from the first AM106 administration and then once every 3 days until sacrificed. Control mice received similar amounts of purified human IgG only.

### Tissue samples and histology.

Eight weeks after the first administration of Dox, animals were sacrificed, and their hearts were arrested in diastole by intravenous injection of 0.2 ml 10% potassium chloride (MERCK, USA). The heart was excised and rinsed with ice cold DPBS before the removal of the right ventricle and the atria. For the histopathological analyses, papillary slices of the left ventricle of seven mice from each treatment group were fixed in 4% formaldehyde up to 24 h before paraffin embedding. FITC-labeled wheat germ agglutinin (WGA, LIFE TECHNOLOGIES, 1:200 dilution) staining was performed to detect cardiomyocyte cross sectional area in the left ventricular myocardium. The cardiac myocyte membranes were observed by fluorescence microscopy. The morphometric analyses were performed using IMAGE-PRO PLUS software. Only cells with well-defined cell membranes were selected. At least one hundred myocytes were analyzed in each group. Sirius red staining was performed to evaluate fibrosis. For its quantification, at least six random pictures from the remote non-infarcted zone were taken from each slide at 20x magnification. Collagen deposition was used to define fibrosis, which is expressed as a percentage of red pixels to green pixels quantitated using DIGITAL IMAGE HUB software version 4.0.6. For other molecular-cellular biological studies, tissue from the left ventricular myocardium was collected and stored at - 80 °C for RNA extraction and Western blot. The observers who performed the images analyses, and the molecular and cellular biological experiments were blinded to the experimental groups.

### Immunohistochemistry.

The next experimental procedure was performed as previously described, with some modifications. Briefly, the sections (3 µm) from paraffin-embedded papillary slices of LV were placed on poly-I-lysine-coated glass slides. Then, the sections were de-paraffinized and pretreated in DAKO PT Link for 20 min at 97 ºC. For YY1 or HDAC4 staining, rehydrated sections were incubated overnight with a polyclonal YY1 antibody (working dilution 1:500, PROTEINTECH, Chicago, IL, USA) or with a polyclonal HDAC4 antibody (working dilution 1:500, PROTEINTECH, Chicago, IL, USA). Next, the sections were incubated with anti-rabbit biotinylated-labelled polymer (DAKO ENVISION) according to the manufacturer's instructions and revealed with 2-2'diaminobencidine (DAB). A cytoplasmic dark-brown precipitate indicated a positive immunostaining. Images were captured using a Zeiss Axio Scope A10 (CARL ZEISS, Madrid, Spain) microscope. Six random pictures were taken of each slide at 10× magnification (n = 7 slices/each treated group).

### Levels of c-TnT, LDH, MDA and sST2 in serum and cell culture media.

The concentrations of each one cited molecule in serum and in the cellular supernatant were measured using ELISA procedure. Lactate Dehydrogenase Kits to human (MBS009535), mouse (MBS2022204) as well as ELISA kits to measurement the levels of sST2 or c-Tnt (MBS262307) were obtained from MYBIOSOURCE.COM. From abcam we obtained MDA Assay Kit (ab238537). In all cases, the reaction was terminated using a stop solution, and the absorbance was determined at 450 nm using a microplate reader (CLARIOstar, BMG LABTECK, Ortenberg, Germany). The intra and inter-assay precisions globally were ≤ 10%. RNA isolation and quantitative RT-PCR. Total RNA was isolated from hiPsCMs (2×10⁶ cells) or from left ventricular myocardium (20 mg). RNA was purified with the RNeasy Mini Kit (QIAGEN), and cDNA was prepared with the iScript cDNA Synthesis Kit (BIORAD LAB. INC., Madrid) according to the manufacturer's recommendation. Quantitative real time polymerase chain reaction (RT-qPCR) was performed with the TB Green Premix Ex Taq II (Tli RNase H Plus) Master Mix (TAKARA BIO INC., Europe). Glyceraldehyde 3-phosphate dehydrogenase (GAPDH) was used as housekeeping gene. Sequences of the used primers (MERCK, USA) are listed in Table 1.

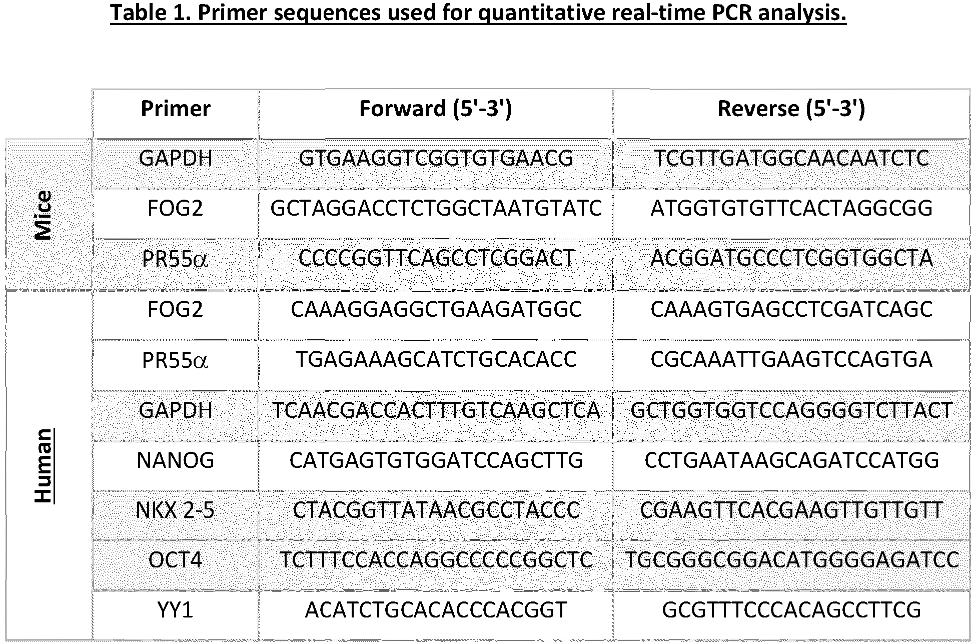

Total RNA was isolated from hiPsCMs (2×10⁶ cells) or from left ventricular myocardium (30 mg) and reverse-transcribed to cDNA using the miRCURY LNA Universal RT microRNA cDNA synthesis kit (Qiagen). Mature miR-106b expression levels were assessed by quantitative RT-PCR using the miRCURY LNA UniRT PCR primer for miR813 106b-5p (Qiagen) and Power SYBRâGreen PCR Master Mix (Thermo Fisher Scientific). U6 (Qiagen) was used as internal control to normalize miR-106b expression.

### Subcellular fractionation.

Briefly, fresh LV (^{∼} 30 mg) tissue or harvested untreated or treated cells (^{∼}3 × 10⁶ cells) were washed using cold DPBS along with 100-fold diluted protease and phosphatase inhibitors. Human cardiomyocytes were pelleted by centrifugation at 480 xg for 10 min at 4 °C, while the myocardium was placed in a pre-chilled glass Petri dish and minced on ice using sharp scissors. All samples were homogenized in 500 µl of STM buffer at pH 7.4, containing 250 mM sucrose, 50 mM Tris-HCI, 5 mM MgCI2, 5 mM Na3VO4, 1% (v/v) protease and phosphatase inhibitor cocktails and homogenized for 1 min on ice using a tight-fitting Teflon pestle attached to a Potter S. homogenizer (Sartorius Stedium, Goettingen, Germany) set to 800 rpm. The homogenate was maintained on ice for 30 min, vortexed at maximum speed for 15 s and then centrifuged at 800 xg for 15 min at 4 °C. The pellet was labelled as P0 and kept on ice; the supernatant was labelled as S0. The pellet P0 (containing nuclei and debris) was resuspended in 500 µl STM buffer, vortexed at maximum speed for 15 s and then centrifuged at 500 xg for 15 min at 4 °C. Following the above step, the nuclear pellet was labelled as P1 and kept on ice, and the supernatant S1 (cell debris) was discarded. To further increase the purity of the P1 fraction it was washed in 400 µl STM buffer, vortexed at maximum speed for 15 s and then centrifuged at 1000 xg for 15 min at 4 °C. The washed pellet was labelled as P2 (S2 was discarded) and resuspended in 100 µl NET buffer composed of 20 mM Hepes pH 7.9, 1.5 mM MgCI2, 0.5 M NaCl, 0.2 mM EDTA, 20% (v/v) glycerol, 1% (v/v) Triton-X-100 and protease and phosphatase inhibitors, using a pipette to triturate until homogeneity. Pellet P2 was vortexed at maximum speed for 15 s and incubated on ice for 30 min; the resulting fraction contained the nuclei, which were lysed by sonication (Soniprep 150, MSE, London, UK) at a high setting for 10-15 s with 30 s pauses whilst being kept on ice. The lysate was centrifuged at 9000 xg for 30 min at 4 °C, the resultant supernatant (S3) was the final "nuclear fraction". Cytosolic fractions were extracted from S0 by centrifugation at 11,000 xg for 10 min at 4 °C. The supernatant contained the cytosol fraction. The protein content of each compartment was determined using the BCA protein assay⁴⁸. To analyze total protein fraction, left ventricular samples or cells were collected, washed, and lysed with a RIPA solution (THERMOFISHER, USA) supplemented with protease and phosphatase inhibitors (1:100).

### Western blotting.

Protein extracts (10 µg) obtained were denatured, separated by SDS-PAGE electrophoresis (Bio-Rad), transferred onto PVDF membrane (Millipore) and probed with antibodies to GADPH (MERCK, 8795,1:5000), TBP (CELL SIGNALING, 8515, 1:1000), PR55α (CELL SIGNALING, 5689, 1:1000), phospho-HDAC4 (ABCAM, ab39408, 1:2000), HDAC4 (CELLSIGNALING, 5392, 1:1000), YY1 (CELL SIGNALING, 2185,1:1000) and sST2 (PROTEINTECH, 11,920-1-AP, 1:1000). Membranes were developed using HRP conjugated secondary antibodies and Pierce ECL western blotting substrate (Thermo Fisher Scientific). Signals were detected by chemiluminescence and quantified using ImageJ 1.44p pixel analysis (US National Institutes of Health).

### Co-immunoprecipitation protocol.

For co-immunoprecipitation, each extract from the cells (8 × 10⁶ cells) or from the left ventricular area (400 µg) were collected and incubated overnight only in the presence of an antibody to HDAC4 (CELL SIGNALING, 5392, 1:100) or YY1 (CELL SIGNALING, 2185, 1:500) at 4 °C with gentle shaking. After this, 20 µl of protein A-Sepharose slurry were added and samples were incubated for 4 h at 4 °C with gentle shaking. Then, beads were washed three times with IP buffer (20 mM HEPES pH 7.4, 0.5 mM EDTA, 150 mM NaCl and 0.1% Triton X-100) to remove non-specific binding. For each wash, the beads were mixed gently with IP buffer, centrifuged at 4 °C and the supernatant was discarded. The antigen-antibody complex is eluted from the beads by heating samples in 25 µl 1× SDS gel loading buffer for 4 min. To analyze the immunoprecipitate by western blot, samples were loaded into a continuous SDS-PAGE gel and run at 30 mA constant current for 2 h. The interaction was analyzed by western blotting using primary antibodies to HDAC4 (CELL SIGNALING, 5392, 1:1000) or YY1 (CELL SIGNALING, 2185, 1:1000). As control, we used IgG.

### Luciferase reporter assay.

To confirm that miR-106b-5p targets the 3'-UTR of PR55α, we cloned the region of PR55α 3'-UTR, which contains the predicted miR-106b binding sites, into a pGL14 Luciferase reporter vector (Luc-PR55α-WT; PROMEGA, Madison, WI, USA). Luc PR55α-mutant (Luc-PR55α-Mut) was also constructed, which contained a mutated PR55α at the binding site of miR-106b (5'-GCACUUU-3' mutated to 5'- CGUUUAAA-3') using In-Fusion cloning kit (CLONTECH, Mountain View, CA, USA) as per manufacturer's protocol. Dual-Luciferase Reporter Assay System was applied for luciferase reporter assay (PROMEGA). HEK 293T/17 cells (ATCC) was simultaneously transfected with wild type (wt) or mutant (mut) type 3'-UTR PR55α and miR-106b mimic or NC mimic. Luciferase activity was detected using CytoFLEX Flow Cytometer (BECKMAN COULTER). Firefly luciferase activity was normalized to Renilla luciferase activity. The ratio of PR55α 3'-UTR to NC mimic was set as 1; therefore, the displayed results indicate the fold changes relative to control. The experiments were performed three times in triplicate.

### Statistical analyses.

Data obtained were reported as mean ± SEM. Statistical differences were evaluated by fitting linear models with interactions (determined by two-way ANOVA followed by Bonferroni's post hoc test) and estimating marginal means. Holm correction was used for multiple comparisons. A p value < 0.05 was considered significant.

### Results

### MiR-106b-5p promotes Dox-induced myocardial apoptosis

First, to evaluate whether miR-106b-5p takes part in the complex molecular mechanism involved in anthracyclines-induced chronic heart failure, we assessed its modulation in iPS cell-derived human cardiomyocytes (hiPsCMs) exposed to 5 µM doxorubicin (Dox); a translational platform. Data analysis by quantitative real time PCR revealed a progressive upregulation in miR-106b levels following Dox treatment (Figure 1a). In particular, we evaluated a time-dependent increase in miR-106b levels, which was clearly visible after 3 h of treatment (2.3±0.03-fold control; figure 1a) and significantly higher following 15 h (p<0.001). Furthermore, the miR-106b levels were progressively increased in the culture media of hiPsCMs, being significantly from 15 h of Dox treatment (p<0.001) (Figure 1b). Based on these results, we hypothesized that miR-106b levels are increased in hiPsCMs under Dox treatment.

In addition, data analysis allowed us selected 15 h as the time of treatment with Dox for the following tests.

Next, to validate the potential roles of miR-106b in Dox-induced CTX, we next sought to inhibit miR-106b by using chemically modified antisense oligonucleotides to block specifically the miR-106b. Therefore, the efficiency of miR-106b inhibition was tested by assessing the expression level of a known miR-106b target, FOG. In comparison with the antagomir negative control (AC), antimiR-106b (AM106) added in preincubation for 24 h was able to prevent down-regulation of FOG2 mRNA levels when hiPsCMs were treated with Dox for 15 h (**figure 7**). Further, AM106 therapy was able to prevent the release of LDH, the loss of Dox-induced cell viability and significantly alleviated Dox-induced myocardial apoptosis as documented by a decrease in caspase 3/7 activity (Figure 1, c-e). In order to demonstrate whether miR-106b participates in regulation the sensitivity of cardiomyocytes to Dox, low dose of Dox (0.2 µM) was used to treat cardiomyocytes. Overexpression of miR-106b using a miR-106b mimic (Figure 1, f-h) sensitized hiPsCMs to cell death in term of a loss of cellular viability and an increase in 3/7 caspase activity (Figure 1, f-g).

**Taken together, our results suggest for first time that human cardiomyocytes under Dox treatment are able to increase miR-106b levels, which was associated with the Dox-induced CTX.**

### PR55α is a downstream target of miR-106b.

In an effort to explore the molecular mechanism by which miR-106b is involved in Dox-induced CTX, we evaluated its potential targets using the bioinformatic programs TargetScan, miRTarBase and Enrichr. We identified that regulatory subunit of PP2A (PR55α) has conserved binding sites for miR-106b (Figure 2a). Furthermore, we detected PR55α expression in the hiPsCMs upon Dox treatment and evaluated that PR55α expression was decreased, in a time dependent-manner. As shown in figure 2b, Dox treatment caused a decrease in PR55α protein levels, which was clearly visible after 6 h of treatment (2.6±0.02-fold control) and significantly higher following 15 h (p<0.001). A negative correlation was evaluated between miR-106b levels and PR55α protein levels in hiPsCMs treated with Dox (r=-0.927, p<0.001). Then, we analyzed whether the decreased expression of PR55α was due to the increased of miR-106b, after Dox treatment. We found that inhibition of miR-106b recovered PR55α levels in hiPsCMs exposed to Dox treatment (Figure 2c). As shown in figure 2d, forced expression of miR-106b in hiPsCMs significantly attenuated PR55α levels. When we evaluated the effect of Dox treatment on PP2A activity (Figure 2e), data analysis showed that antimiR 106b therapy prevented Dox-induced PP2A activity decrease (p<0.001) and the use of 106b mimic simulated the effect of Dox.

**Taken together, our results concluded that miR-106b could suppress PR55α expression levels in hiPsCMs under Dox treatment which led to decrease PP2A activity in hiPsCM.**

Next, to demonstrate that miR-106b directly binds to the 3'UTR of PR55α mRNA and downregulates PR55α protein expression, a luciferase gene reporter assay was used (Figure 2, f-h). We constructed two luciferase plasmids carrying the full-length PR55α (Luc-PR55α-WT [wild type]) and the mutated PR55α (Luc-PR55α-Mut) (Figure 2f). Dual luciferase reporter gene assay showed that the relative activity of firefly luciferase of Luc PR55α-WT in HEK293 cells was significantly decreased by co-transfection of miR-106b, whereas it did not change the luciferase activity of Luc-PR55α-Mut (Figures 2, f-g). **On the basis of these findings, our data indicate that PR55α is a direct target of miR-106b.**

**MiR-106b-induced PR55α down-regulation favors cytosol accumulation of HDAC4 acting as a co-repressor of Yy1 transcription factor.**

Several studies have found that histone deacetylase 4 (HDAC4) required the actions of PP2A holoenzyme containing the PR55α subunit for its nuclear import and regulatory activity^{19,20}. As shown in figure 3, panels a-b, phosphorylation state of HDAC4 Ser246 in cytosol from hiPsCMs was evaluated and significantly increases (p<0.001) when cells were treated with Dox for 15h. Preincubation with AM106 for 24h before Dox treatment decreased phosphorylation state of HDAC4 and an overexpression of miR-106b using a miR-106b mimic simulated the effect of Dox (p<0.001). When hiPsCMs were exposed to a specific microRNA Target Site Blocker (PR55α protector) in preincubation before Dox treatment for 24h, and in compared to Dox treated group, phosphorylated HDAC4 Ser246 levels were significantly reduced (p<0.001). Interestingly, when hiPsCMs were co-transfected with AM106 plus PR55α siRNA before Dox treatment for 24h, AM106 therapy was no able to prevent Dox-induced HDAC4 phosphorylation on its activating site Ser246. Next, we evaluated total HDAC4 protein levels both in cytosol as well as in nuclei extracts and we analyzed data as ratio HDAC4 nuclei/cytosol (Figure 3). Our results determined a significative decrease in total HDAC4 levels in nuclei fractions obtained from hiPsCMs exposed to Dox (p<0.001), which increased when hiPsCMs were pretreated with AM106 (p<0.001) before Dox treatment. Moreover, 106b-mimic decreased total HDAC4 protein levels (p<0.001) in the nuclear fractions from hiPsCMs, the used of a PR55α protector significantly increased its nuclear level (p<0.001) even in presence of Dox and co-transfection with AM106 plus PR55α siRNA before Dox treatment was able to reduce total HDAC4 levels (p<0.001) in nuclear fraction.

**Taken together, these results suggest that miR-106b through PR55a degradation prevents dephosphorylation HDAC4, which leaded to accumulation of phosphorylated HDAC4 in cytosolic fraction.**

Once we identified that PR55α is a direct target of miR-106b and confirmed its relationship with HDAC4, the next step was to assess both the involvement of the HDAC4 on anthracycline-induced CTX as well as the molecular mechanisms implicated. Interactome analysis confirmed that HDAC4 was able to interact with YY1 transcription factor in the human heart with a score of 0.93 (**figure 8**). In this setting, the next step was to evaluate the effect of Dox on YY1 protein levels. When hiPsCMs were exposed to Dox for various prolonged times, YY1 protein expression increased in a time-dependent manner, which was clearly visible after 6 h of treatment (3.8±0.23-fold control; figure 4a) and significantly higher following 15 h (p<0.001).

Then, and with the global aim to test experimentally whether YY1 was able to interact with HDAC4, co-immunoprecipitation assays were carried out (Figure 4, panels b-d). Initially, nuclear fraction from hiPsCMs under different experimental conditions were immunoprecipitated with an HDAC4 antibody and probed with an anti-YY1 antibody. In parallel, cited lysates were immunoprecipitated with an anti-YY1 antibody and probed with an anti-HDAC4 antibody. This reciprocal immunoprecipitation experiment showed that Dox was able to prevent HDAC4-YY1 interaction whereas antimiR 106b favored these interaction (Figure 4, b-d). Furthermore, overexpression of miR-106b using a miR-106b mimic simulated the effect of Dox and the use of PR55α protector prevented miR 106b effect in presence or not of Dox (Figure 4, b-d).

Based on these and other results previously obtained by the inventors using non oncological experimental models with HF, the next step was to study whether the cardioprotective effect attributed to AM106 against Dox-induced CTX was related to modulation of sST2 isoform (Figure 4e and **figure 9**) by the transcription factor YY1. In a first approach, in this study it was evaluated an increase in sST2 expression levels when hiPsCMs were exposed to Dox for 15 h (Figure 4e and **figure 9a**). As it shown in figure 4e, AM106 therapy reverted this effect, miR-106b mimic simulated the effect of Dox and PR55α protector in absence or not of Dox decreased sST2 expression (p<0.001, in all cases) (Figure 4e, left). Furthermore, the specific silencing of PR55α together with functional blockade of miR106b (Figure 4e, right) or conversely silencing of endogenous YY1 expression (figure 9) allowed us to: (1) Characterize the involvement of the miR106b/PR55α axis on sST2 expression; and (2) Evaluate the role of YY1 on sST2 expression and its effect on cell viability. In this context, silencing of PR55α reversed the effect of AM106 on sST2 expression in Dox-treated hiPsCMs cells (Figure 4e) and silencing of YY1 prevented the effect of Dox on sST2 levels (**figure 9a**) which leaded to improved cell viability (**figure 9b**).

Taken together our data suggests that Dox was able to increase sST2 levels through modulation of the miR106b/HDAC4/YY1 axis, and that this increase is related to anthracycline-induced CTX.

Once we confirmed that Dox-induced sST2 overexpression in hiPsCMs was associated with a decrease in HDAC4-YY1 interaction and the role of miR-106b, the next step was to evaluate the implication of PR55α using a viral delivery strategy to overexpress hPR55α in hiPsCMs. First the efficiency of transduction was tested by infecting hiPsCMs with AAV6-EGFP viral particles at a MOI of 10⁴. After 7 days, more than 80% of the hiPsCMs expressed EGFP, as analyzed by immunostaining. Following this, AAV6-hPR55α and AAV6-empty (AAV6-Ctrl) particles were produced to infect hiPsCMs again at a MOI of 1 × 10⁴. The AAV6-hPR55α transduction resulted in a significant and stable induction of hPR55α mRNA expression in 7 days (Figure 4f). Dox strongly induced an upregulation of sST2 levels in AAV6-Ctrl treated cells, whereas cells treated with AAV6-hPR55α showed a significant decrease in sST2 levels (Figure 4g). To gain further insights into the role of sST2 to anthracycline-induced CTX, we next examined whether incubation with the soluble extracellular domain of ST2 coupled to the Fc fragment of human IgG1 (sST2/Fc) could interfere with the cardioprotective effect of AM106 therapy.

As showed in **figure 10**, exogenous sST2/Fc treatment was able to prevent the cardioprotective effect of AM106 in term of LDH release and cell viability (p<0.001 in all cases).

**AntimiR-106b therapy** protects **from doxorubicin-induced cardiotoxicity in vivo.**

Once we identified the cardioprotective effect of AM106 therapy against Dox-induced CTX in a translational platform, the next step was tested whether this therapy was able to prevent Dox-induced cardiac dysfunction in an established experimental animal model. Based on our obtained in vitro results, we speculated that in vivo blocking of miR-106b could was able to prevent cardiotoxic effects of Dox due to decrease of myocardial sST2 protein expression. Thus, to achieve a systemic loss of function of miR-106b, we injected AM106 (20 mg/kg, i.v.) to the mice seven days before the first Dox injection (5 mg/kg, i.p.). Next, mice were injected for five consecutive weeks, and simultaneously, with 5 mg/kg Dox and 20 mg/kg AM106 and eight weeks later sacrificed (Figure 5a). As shown in figure 5, and similarly to pioneer data obtained in hiPsCMs, AM106 therapy not only induced a significant up-regulation of FOG2 and PR55α mRNA levels in presence of Dox (p<0.001, in all cases) (Figure5b), but also it was able to prevent Dox-induced CTX. AM106 therapy decreased both cardiac troponin T (cTnT) levels, LDH activity as well as sST2 plasma levels (p<0.001, in all cases) (Figure 5c). Further, Dox treatment induced significant cardiac atrophy and fibrosis after the cumulative 25 mg/kg dose (Figure 5d). Importantly, the reduction in heart weight, cardiomyocyte size and cardiac fibrosis were completely inhibited by pretreatment with AM106 (p<0.001, in all cases) (Figure 5d). In addition, Dox showed a significant decline in ejection fraction (EF) and fractional shorting (FS), which were prevented by AM106 therapy (p<0.001, in both cases) (Figure 5e and **figure 13**).

Dox treatment also induced cellular apoptosis, as detected by TUNEL staining and activation of 3/7 caspase activity, which were prevented by antimiR-106b therapy (p<0.001, in all cases) (**figure 11a**). Dox treatment also resulted in whole-body cachexia, mainly due to reduced muscle mass and antimiR-106b inhibited the loss of body mass (p<0.001) (**figure 11b**). Further, antimiR-106b therapy was able to prevent the mortality associated with Dox treatment (p<0.001) (**figure 11c**).

### Downstream effectors of miR-106-5p

As expected, AM106 therapy had no effect on the Dox-induced YY1 increase (p<0.001) nor on the Dox-induced HDAC4 increase (Figure 6a). Further, AM106 was able to prevent the effect of Dox on YY1-HDAC4 interaction (p<0.001) and decreased sST2 expression (p<0.001) in LV myocardium (Figure 6, b-c). Again, mice treated with PR55α protector showed a similar response pattern to the use of AM106 (p<0.001) (Figure 6b). Finally, to obtain more direct evidence that the beneficial effect of AM106 therapy was related to down regulation of the myocardial sST2 expression, we next examined whether the use of soluble sST2/Fc protein could interfere with the cardioprotective effect of AM106 therapy. As shown in Figure 6c, sST2/Fc treatment was able to prevent the cardioprotective effect of AM106, in term of c-TnT, LDH and MDA levels (p<0.001, in all cases)

Our results demonstrate that PR55α is highly involved in miR-106b dependent regulation of Dox-induced CTX, and its degradation might be one of the mechanism responsible for the loss of cardiac function, as seen in the case of the Dox-stress model applied. In fact, we herein demonstrate that Dox-induced miR-106b upregulation reduces cardiac PR55α levels leading to loss of PP2A phosphatase activity and accumulation of phosphorylated HDAC4 in the cytosol. Therapy with AM106 prevents this signaling axis by favouring: (1) dephosphorylation of HDAC4 and its accumulation in the cell nucleus; (2) the interaction between HDAC4 and YY1 in nuclei; and (3) down-regulation of the sST2 isoform.

## Claims

1. An oligonucleotide and/or oligonucleotide analogue molecule which is an antagonist of the miRNA-106b-5p, for use in a method to treat or prevent one or more symptoms of the cardiotoxicity that is developed in oncologic patients after receiving anthracycline-based chemotherapy.

2. The oligonucleotide and/or oligonucleotide analogue molecule for use according to claim 1, where the method is to treat or prevent the loss of muscle mass and cardiac atrophy that is developed in the oncologic patients after receiving anthracycline-based chemotherapy.

3. The oligonucleotide and/or oligonucleotide analogue molecule for use according to any of claims 1 to 2, wherein the method prevents, reduces or mitigates the myocardial dysfunction in oncologic patients after receiving anthracycline-based chemotherapy.

4. The oligonucleotide and/or oligonucleotide analogue molecule for use according to any of claims 1 to 3, wherein the oligonucleotide and/or oligonucleotide analogue molecule is administered systemically to the oncologic patient.

5. The oligonucleotide and/or oligonucleotide analogue molecule for use according to any of claims 1 to 3, wherein the anthracycline-based chemotherapy is doxorubicin.

6. The oligonucleotide and/or oligonucleotide analogue molecule for use according to any of claims 1 to 5, wherein the oligonucleotide and/or oligonucleotide analogue molecule is at least 85% complementary to the full length sequence of the nitrogenous bases of human miR-106b/5p as represented in SEQ ID NO 1.

7. The oligonucleotide and/or oligonucleotide analogue molecule for use according to any of claims 1 to 5, wherein the oligonucleotide and/or oligonucleotide analogue molecule is at least 95% complementary to the full-length sequence of the nitrogenous bases of human miR-106b/5p as represented in SEQ ID NO 1.

8. The oligonucleotide and/or oligonucleotide analogue molecule for use according to any of claims 1 to 7, wherein the oligonucleotide and/or oligonucleotide analogue molecule is an antagomiR, an antimiR or a microRNA sponge.

9. The oligonucleotide and/or oligonucleotide analogue molecule for use according to claim 8, wherein the oligonucleotide and/or oligonucleotide analogue molecule is an antimiR.

10. The oligonucleotide and/or oligonucleotide analogue molecule for use according to claim 9, wherein the oligonucleotide and/or oligonucleotide analogue molecule is an antimiR whose sequence is a fragment composed of a succession of at least 10 consecutive nitrogen bases of nucleotide or nucleotide analogue units that are identical in at least 85% to the complementary sequence of a region as present in miRNA-106b-5p of SEQ ID NO 1.

11. The oligonucleotide and/or oligonucleotide analogue molecule for use according to claim 9, wherein the oligonucleotide and/or oligonucleotide analogue molecule is an antimiR whose sequence is composed of a succession of at least 10 consecutive nitrogen bases of nucleotide or nucleotide analogue units that are identical in at least 95% to the complementary sequence of a region as present in miRNA-106b-5p of SEQ ID NO 1.

12. The oligonucleotide and/or oligonucleotide analogue molecule for use according to claim 9, wherein the oligonucleotide and/or oligonucleotide analogue molecule is an antimiR whose sequence is composed of a succession of at least 15 consecutive nitrogen bases of nucleotide or nucleotide analogue units that are identical in at least 100% to the complementary sequence of a region as present in miRNA-106b-5p of SEQ ID NO 1.

13. The oligonucleotide and/or oligonucleotide analogue molecule for use according to claim 9, wherein the oligonucleotide and/or oligonucleotide analogue molecule consists of SEQ ID NO 3.

14. The oligonucleotide and/or oligonucleotide analogue molecule for use according to any of claims 10 to 13, wherein at least one of the nucleotides comprised in said oligonucleotide and/or oligonucleotide analogue molecule is chemically modified, and wherein said chemically modification is selected from the group of:
i) 2'-O-methyl (2'OMe),
ii) 2'-O-Methoxyethyl (2' MOE), and/or
iii) an extra bridge connecting the 2' oxygen and 4' carbon (LNA).

15. A composition, preferably a pharmaceutical composition, comprising at least an oligonucleotide and/or oligonucleotide analogue molecule as defined in any of claims 1 to 14, or a mixture of two or more of them, optionally further comprising a carrier and/or one or more pharmaceutically acceptable excipients.
